# EUROPÄISCHE PATENTSCHRIFT

(11) **EP 1 263 818 B1**
(45) Veröffentlichungstag und Bekanntmachung des Hinweises auf die Patenterteilung: **16.06.2004**
(21) Anmeldenummer: 01907554.8
(22) Anmeldetag: 22.02.2001
(51) Int. Cl.: C08F 220/54, A61K 7/06, C08F 220/18

(54) **KOSMETISCHES MITTEL**
COSMETIC AGENT
PRODUIT COSMETIQUE

(30) Priorität: 23.02.2000 DE 10008263
(43) Veröffentlichungstag der Anmeldung: 11.12.2002
(73) Patentinhaber: BASF AKTIENGESELLSCHAFT, 67056 Ludwigshafen (DE)
(72) Erfinder: NGUYEN KIM, Son, 69502 Hemsbach (DE); WOOD, Claudia, 69469 Weinheim (DE)
(74) Vertreter: Kinzebach, Werner, Dr.
(86) Internationale Anmeldenummer: PCT/EP2001/002047
(87) Internationale Veröffentlichungsnummer: WO 2001/062809

(56) Entgegenhaltungen:
- EP-A- 0 373 442
- WO-A-96/19966
- DE-A- 19 838 196

## Beschreibung

Die vorliegende Erfindung betrifft ein kosmetisches Mittel, welches wenigstens ein wasserlösliches oder wasserdispergierbares Polymer einpolymerisiert enthält.

In der Kosmetik werden Polymere mit filmbildenden Eigenschaften zur Festigung, Strukturverbesserung und Formgebung der Haare verwendet. Diese Haarbehandlungsmittel enthalten im Allgemeinen eine Lösung des Filmbildners in einem Alkohol oder einem Gemisch aus Alkohol und Wasser.

Haarfestigungsmittel werden im Allgemeinen in Form von wässrigalkoholischen Lösungen auf die Haare aufgesprüht. Nach dem Verdampfen des Lösungsmittels werden die Haare an den gegenseitigen Berührungspunkten vom zurückbleibenden Polymer in der gewünschten Form gehalten. Die Polymere sollen einerseits so hydrophil sein, dass sie aus dem Haar ausgewaschen werden können, andererseits aber sollen sie hydrophob sein, damit die mit den Polymeren behandelten Haare auch bei hoher Luftfeuchtigkeit ihre Form behalten und nicht miteinander verkleben. Um eine möglichst effiziente Haarfestigerwirkung zu erzielen, ist es außerdem wünschenswert, Polymere einzusetzen, welche ein relativ hohes Molekulargewicht und eine relativ hohe Glastemperatur (mindestens 15 °C) besitzen.

Ein weiterer aktueller Anspruch an Haarbehandlungsmittel ist es, eine gute Festigungswirkung zu erzielen, auch dann, wenn das Mittel bei Personen eingesetzt wird, die von Natur aus besonders kräftiges und/oder dunkles Haar haben. Dabei soll dem Haar ein möglichst natürliches Aussehen und Glanz verliehen werden.

Ein Nachteil vieler bekannter Haarfestigerpolymere ist der sogenannte "Flaking"-Effekt, d. h. nach dem Auskämmen bleibt ein weißer, schuppenförmiger Rest auf dem Haar zurück. Dies wird von den Anwendern im Allgemeinen als äußerst unangenehm empfunden. Der "Flaking"-Effekt tritt besonders deutlich bei Personen mit dunkler Haarfarbe und/oder besonders kräftigen Haaren auf. Die Einsatzmöglichkeit von Haarfestigerformulierungen, die diesen Effekt aufweisen, ist somit insbesondere auf dem asiatischen Markt deutlich beeinträchtigt. Als mögliche Ursache für den "Flaking"-Effekt werden u. a. die chemische Struktur der eingesetzten Haarfestigerpolymere und insbesondere die Partikelgröße des Sprays angesehen.

Neben den zuvor genannten Eigenschaften sollen die in den Mitteln eingesetzten Haarfestigerpolymere daher vorzugsweise eine hohe Treibgasverträglichkeit aufweisen, um eine Formulierung in Spraydosen unter möglichst hohem Druck zuzulassen. Dies gilt sowohl für die klassischen Treibmittel auf Propan/Butan-Basis, als auch für deren Ersatzstoffe, z. B. auf Dimethyletherbasis.

Es ist bekannt, Copolymerisate auf Basis von Monomeren mit kationischen oder kationogenen Gruppen in Haarbehandlungsmitteln einzusetzen. So beschreiben die US 3,914,403 und die US 3,954,960 Haarkosmetika, die Copolymerisate enthalten, welche N-Vinylpyrrolidon, ein Monomer mit einer quaternisierbaren Gruppe sowie gegebenenfalls weitere Monomere eingebaut enthalten.

Die WO-A-96/19966 und die WO-A-96/20227 beschreiben Haarbehandlungsmittel auf Basis von Terpolymeren, die ein Vinyllactam, ein Acrylat oder Acrylamid mit einer quaternären Aminogruppe und ein weiteres hydrophobes Monomer, bevorzugt ein C₄- bis C₃₂-Alkyl(meth)acrylat oder -(meth)acrylamid, eingebaut enthalten. Die Propan/Butan-Verträglichkeit der in diesen Mitteln eingesetzten Polymere ist noch verbesserungswürdig.

Die WO-A-96/19967 beschreibt ein Verfahren zur Herstellung von Copolymerisaten aus Vinylpyrrolidon und N-3,3-Dimethylaminopropylmethacrylamid und deren Einsatz in Haarbehandlungsmitteln. Die WO-A-96/19971 beschreibt Haarsprayformulierungen mit einem geringen Gehalt an flüchtigen organischen Verbindungen (VOC-Gehalt), die ein Terpolymer aus Vinylpyrrolidon, Vinylcaprolactam und N-3,3-Dimethylaminopropylmethacrylamid (DMAPMA) enthalten. Die WO-A-96/20694 beschreibt eine Haarsprayformulierung mit einem geringen VOC-Gehalt, die ein Tetramer aus Vinylpyrrolidon, Vinylcaprolactam, DMAPMA und einem C₈- bis C₁₈-Alkylacrylamid oder -acrylat enthält. Polymere, die einen höheren Anteil langkettiger Acrylat- und/oder Acrylamidmonomere eingebaut enthalten, führen in der Regel zu sehr weichen Filmen. Haarbehandlungsmittel, die solche Polymere enthalten, sind im Allgemeinen verbesserungswürdig im Hinblick auf ihre Festigungswirkung.

Nachteilig an den zuvor genannten Mitteln auf Basis von Polymeren mit einem hohen Vinyllactamanteil ist, dass diese klebrig sind und bei hoher Luftfeuchtigkeit leicht an Festigungswirkung verlieren. Keines der zuvor genannten Dokumente beschreibt den Einsatz von Polymeren, die wenigstens einen tert.-Butylester oder ein N-tert.-Butylamid einer α,β-ethylenisch ungesättigten Carbonsäure eingebaut enthalten, in der Haarkosmetik.

Die EP-A-0 372 546 und die EP-A-0 728 778 beschreiben Filmbildnerharze, welche wenigstens ein (Meth)acrylamid, wenigstens ein C₁- bis C₄-Alkyl(meth)acrylat, wenigstens ein N,N-Dialkyl(meth)acrylat oder N,N-Dialkyl(meth)acrylamid und gegebenenfalls wenigstens ein Hydroxyalkyl(meth)acrylat oder Polyalkylenglykol(meth)acrylat einpolymerisiert enthalten. Diese Copolymere weisen nur eine geringe Löslichkeit in Ethanol auf, und die resultierenden Filme sind hart, so dass bei ihrer Verwendung in Haarfestigern dem Haar kein natürliches Aussehen verliehen wird. Auch ihre Propan/Butan-Verträglichkeit ist verbesserungswürdig. Nachteilig am Einsatz von Copolymeren, die Hydroxyalkyl(meth)acrylate und/oder Polyalkylenglykol(meth)acrylate eingebaut enthalten, ist weiterhin die hohe Viskosität und die Klebrigkeit darauf basierender wässrigen Präparate.

Anionische Polymere mit Propan/Butan-Verträglichkeit sind bereits bekannt, unter anderem Polymere auf Basis von tert.-Butylacrylat bzw. tert.-Butylmethacrylat.

Die EP-A-379 082 beschreibt z. B. ein Haarfestigungsmittel, enthaltend als Filmbildner ein Copolymerisat, welches
A) 75 bis 99 Gew.-% tert.-Butyl(meth)acrylat,
B) 1 bis 25 Gew.-% (Meth)acrylsäure und
C) 0 bis 10 Gew.-% eines weiteren radikalisch copolymerisierbaren hydrophoben Monomeren
einpolymerisiert enthält. Haarfestigungsmittel auf Basis dieser Copolymere, die nur die Komponenten A) und B) enthalten, machen das Haar zu hart und weisen eine zu geringe Propan/Butan-Verträglichkeit auf. Copolymere, die zusätzlich ein Monomer C) enthalten, sind bezüglich ihrer Auswaschbarkeit verbesserungswürdig.

Die DE-A-43 14 305 beschreibt wie die EP-A-379 082 ein Haarfestigerpolymer auf Basis von tert.-Butyl(meth)acrylat und (Meth)acrylsäure, welches 0 bis 60 Gew.-% eines C₁- bis C₁₈-Alkyl(meth)acrylats oder einer Mischung davon mit N-C₁- bis - C₁₈-Alkyl(meth)acrylamiden einpolymerisiert enthält. Zusätzliche Monomere mit einer Kohlenstoffzahl von mehr als 8 führen zwar unter Umständen zu einer besseren Propan/Butan-Verträglichkeit, wobei jedoch gleichzeitig die Auswaschbarkeit deutlich verschlechtert wird.

Nachteilig an anionischen Polymeren ist zudem, dass die auf ihnen basierenden Haarbehandlungsmittel häufig den zuvor beschriebenen "Flaking"-Effekt aufweisen.

Die deutsche Patentanmeldung DE-A-198 38 196 beschreibt kationische Polymerisate, die durch radikalische Copolymerisation von
(a) 50 bis 70 Gew.-% eines oder mehrerer Monomeren der Formel I 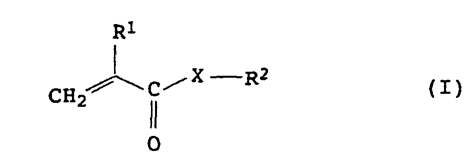
   - X =: O, NR¹,
   - R¹ =: H, C₁-C₈-Alkyl,
   - R² =: tert.-Butyl,
(b) 5 bis 45 Gew.-% eines oder mehrerer Monomeren der Formel II 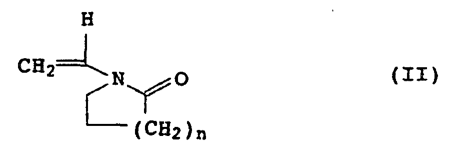 mit n = 1 bis 3,
(c) 5 bis 40 Gew.-% eines monoethylenisch ungesättigten Monomers mit mindestens einer aminhaltigen Gruppe,
erhältlich sind, wobei bis zu 20 Gew.-%, bezogen auf (a), (b) und (c), des Monomeren (a) durch ein Monomer der Formel I mit R² = C₈-C₂₂-Alkyl ersetzt sein können, sowie die Verwendung derartiger Polymerisate in der Haarkosmetik.

Der vorliegenden Erfindung liegt die Aufgabe zugrunde, neue kosmetische Mittel, insbesondere Haarbehandlungsmittel, zur Verfügung zu stellen, die eine hohe Treibgasverträglichkeit aufweisen und/oder die im Wesentlichen keinen "Flaking"-Effekt zeigen. Vorzugsweise sollen diese Mittel eine gute Festigungswirkung aufweisen und dem Haar Glätte und Geschmeidigkeit verleihen. Dabei sollen sie im Allgemeinen eine gute Auswaschbarkeit aufweisen.

Überraschenderweise wurde nun gefunden, dass diese Aufgabe durch kosmetische Mittel gelöst wird, die wenigstens ein wasserlösliches oder wasserdispergierbares Polymer enthalten, das höchstens 50 Gew.-% wenigstens eines tert.-Butylesters und/oder N-tert.-Butylamids einer α,β-ethylenisch ungesättigten Carbonsäure einpolymerisiert enthält.

Gegenstand der vorliegenden Erfindung ist daher ein kosmetisches Mittel, enthaltend wenigstens ein wasserlösliches oder wasserdispergierbares Polymer, das
a) 5 bis weniger als 50 Gew.-% wenigstens eines α,β-ethylenisch ungesättigten Monomers der allgemeinen Formel I 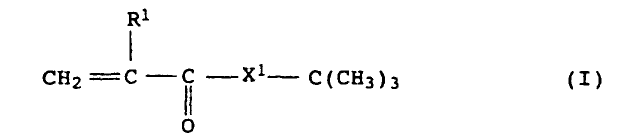 worin
   - R¹: für Wasserstoff oder C₁- bis C₈-Alkyl steht, und
   - X¹: für O oder NR² steht, wobei R² für Wasserstoff, C₁- bis C₈-Alkyl oder C₅- bis C₈-Cycloalkyl steht,
b) 25 bis 90 Gew.-% wenigstens eines N-Vinylamids und/oder N-Vinyllactams,
c) 0,5 bis 30 Gew.-% wenigstens einer Verbindung mit einer radikalisch polymerisierbaren, α,β-ethylenisch ungesättigten Doppelbindung und mindestens einer kationogenen und/oder kationischen Gruppe pro Molekül,
d) 0 bis 30 Gew.-% wenigstens eines α,β-ethylenisch ungesättigten Monomers der allgemeinen Formel II 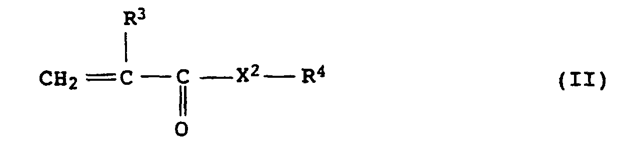 worin
   - R³: für Wasserstoff oder C₁- bis C₈-Alkyl steht,
   - X²: für O oder NR⁵ steht, wobei R⁵ für Wasserstoff, C₁- bis C₈-Alkyl oder C₅- bis C₈-Cycloalkyl steht, und
   - R⁴: für Wasserstoff oder einen linearen C₁- bis C₂₂-Alkylrest steht,
eingebaut enthält, und die Salze davon. Hier und im Folgenden beziehen sich, sofern nichts anderes gesagt wird, alle Angaben in Gew.-% auf die Gesamtmenge aller das Polymer bildenden Komponenten, d. h. die Summe aller Gewichtsanteile der Monomere a), b), c), d) und gegebenenfalls weiterer einpolymerisierter Komponenten beträgt 100 Gew.-%.

Im Rahmen der vorliegenden Erfindung umfasst der Ausdruck C₁- bis C₈-'Alkyl' geradkettige und verzweigte Alkylgruppen mit 1 bis 8 C-Atomen. Vorzugsweise handelt es sich dabei um geradkettige oder verzweigte C₁-C₆-Alkyl- und besonders bevorzugt C₁-C₄-Alkylgruppen. Dazu zählen insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, Octyl etc.

Bei der C₅- bis C₈-Cycloalkylgruppe handelt es sich z. B. um Cyclopentyl, Cyclohexyl, Cycloheptyl oder Cyclooctyl.

Im Folgenden werden Verbindungen, die sich von Acrylsäure und Methacrylsäure ableiten können, teilweise verkürzt durch Einfügen der Silbe "(meth)" in die von der Acrylsäure abgeleitete Verbindung bezeichnet.

### Komponente a)

Bei der Komponente a) handelt es sich bevorzugt um α,β-ethylenisch ungesättigte Verbindungen der allgemeinen Formel I, worin
- R¹: für Wasserstoff, Methyl oder Ethyl steht, und
- X¹: für O oder NR² steht, wobei R² für Wasserstoff, Methyl, Ethyl, n-Propyl, Isopropyl, n-Butyl, sec.-Butyl, tert.-Butyl oder Cyclohexyl steht. Insbesondere steht R² für Wasserstoff.

Dabei können auch Mischungen von Verbindungen der Komponente a) eingesetzt werden.

Bevorzugt handelt es sich bei der Komponente a) um tert.-Butylacrylat, tert.-Butylmethacrylat, tert.-Butylethacrylat, N-tert.-Butylacrylamid, N-tert.-Butylmethacrylamid, N-tert.-Butylethacrylamid und Mischungen davon.

### Komponente b)

Geeignete Monomere b) sind N-Vinyllactame und deren Derivate, die z. B. einen oder mehrere C₁-C₆-Alkylsubstituenten, wie Methyl, Ethyl, n-Propyl, Isopropyl, n-Butyl, sec.-Butyl, tert.-Butyl etc. aufweisen können. Dazu zählen z. B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam etc.

Geeignete Monomere b) sind weiterhin N-Vinylamide, wie N-Vinylformamid, N-Vinylacetamid, N-Vinylpropionamid, N-Vinyl-n-butyramid, N-Vinyl-tert.-butyramid, etc. Bevorzugt wird N-Vinylformamid eingesetzt.

Bevorzugt ist die Komponente b) ausgewählt unter Vinylpyrrolidon, Vinylcaprolactam, Vinylformamid und Mischungen davon.

### Komponente c)

Bevorzugt handelt es sich bei den kationogenen und/oder kationischen Gruppen der Komponente c) um stickstoffhaltige Gruppen, wie primäre, sekundäre und tertiäre Aminogruppen sowie quaternäre Ammoniumgruppen. Vorzugsweise handelt es sich bei den stickstoffhaltigen Gruppen um tertiäre Aminogruppen oder quaternäre Ammoniumgruppen. Geladene kationische Gruppen lassen sich aus den Aminstickstoffen entweder durch Protonierung, z. B. mit Carbonsäuren, wie Milchsäure, oder Mineralsäuren, wie Phosphorsäure, Schwefelsäure und Salzsäure, oder durch Quaternisierung, z. B. mit Alkylierungsmitteln, wie C₁- bis C₄-Alkylhalogeniden oder -sulfaten, erzeugen. Beispiele solcher Alkylierungsmittel sind Ethylchlorid, Ethylbromid, Methylchlorid, Methylbromid, Dimethylsulfat und Diethylsulfat.

Geeignete Verbindungen c) sind z. B. die Ester von α,β-ethylenisch ungesättigten Mono- und Dicarbonsäuren mit C₂- bis C₁₂-Aminoalkoholen, welche am Aminstickstoff C₁- bis C₈-dialkyliert sind. Als Säurekomponente dieser Ester eignen sich z. B. Acrylsäure, Methacrylsäure, Fumarsäure, Maleinsäure, Itaconsäure, Crotonsäure, Maleinsäureanhydrid, Monobutylmaleat und Gemische davon. Bevorzugt werden Acrylsäure, Methacrylsäure und deren Gemische eingesetzt. Bevorzugt sind N,N-Dimethylaminomethyl(meth)acrylat, N,N-Dimethylaminoethyl(meth)acrylat, N,N-Diethylaminoethyl(meth)acrylat, N,N-Dimethylaminopropyl(meth)acrylat, N,N-Diethylaminopropyl(meth)acrylat, N,N-Dimethylaminocyclohexyl(meth)acrylat etc.

Bevorzugt werden N,N-Dimethylaminoethyl(meth)acrylat und N,N-Dimethylaminopropyl(meth)acrylat eingesetzt.

Geeignete Monomere c) sind weiterhin die Amide der zuvor genannten α,β-ethylenisch ungesättigten Mono- und Dicarbonsäuren mit Diaminen, die eine tertiäre und ein primäre oder sekundäre Aminogruppe aufweisen. Dazu zählen z. B. N-[2-(dimethylamino)ethyl]-acrylamid, N-[2-(dimethylamino)ethyl]methacrylamid, N-[3-(dimethylamino)propyl]acrylamid, N-[3-(dimethylamino)propyl]methacrylamid, N-[4-(dimethylamino)butyl]acrylamid, N-[4-(dimethylamino)butyl)methacrylamid, N-[2-(diethylamino)ethyl]acrylamid, N-[4-(dimethylamino)cyclohexyl]acrylamid, N-[4-(dimethylamino)cyclohexyl]methacrylamid etc. Bevorzugt werden N-[3-(dimethylamino)propyl]acrylamid, N-[3-(dimethylamino)propyl]methacrylamid eingesetzt.

Geeignete Monomere c) sind weiterhin N,N-Diallyl-N-alkylamine und deren Säureadditionssalze und Quaternisierungsprodukte. Alkyl steht dabei vorzugsweise für C₁- bis C₂₄-Alkyl. Bevorzugt sind N,N-Diallyl-N-methylamin und N,N-Diallyl-N,N-dimethylammonium-Verbindungen, wie z. B. die Chloride und Bromide.

Geeignete Monomere c) sind weiterhin vinyl- und allylsubstituierte Stickstoffheterocyclen, wie N-vinylimidazol, N-Vinyl-2-methylimidazol, 2- und 4-Vinylpyridin, 2- und 4-Allylpyridin etc.

### Komponente d)

Bevorzugt ist die Komponente d) ausgewählt unter Verbindungen der allgemeinen Formel II 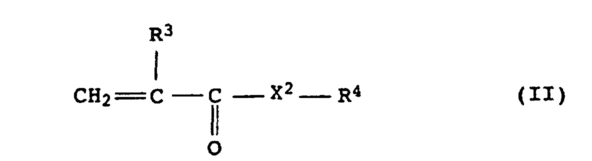 worin
- R³: für Wasserstoff oder C₁- bis C₄-Alkyl steht,
- R⁴: für Wasserstoff oder einen geradkettigen C₁- bis C₃₀-Alkylrest steht, und
- X²: für O oder NR⁵ steht, wobei R⁵ für Wasserstoff, C₁- bis C₈-Alkyl oder C₅- bis C₈-Cycloalkyl steht.

Vorzugsweise steht in der Formel II R³ für Wasserstoff, Methyl oder Ethyl.

Bevorzugt steht X² für O oder NH.

Bevorzugt steht R⁴ für Wasserstoff, Methyl, Ethyl, n-Propyl, n-Butyl, n-Pentyl, n-Hexyl, n-Heptyl, n-Octyl, Undecyl, Lauryl, Tridecyl, Myristyl, Pentadecyl, Palmityl, Margarinyl, Stearyl, Palmit-
oleinyl, Oleyl oder Linolyl.

Vorzugsweise ist die Komponente d) ausgewählt unter n-Butyl(meth)acrylat, n-Octyl(meth)acrylat, n-Nonyl(meth)acrylat, n-Decyl(meth)acrylat, n-Undecyl(meth)acrylat, Tridecyl(meth)acrylat, Myristyl(meth)acrylat, Pentadecyl(meth)acrylat, Palmityl(meth)acrylat, Heptadecyl(meth)acrylat, Nonadecyl(meth)acrylat, Arachinyl(meth)acrylat, Behenyl(meth)acrylat, Lignocerenyl(meth)acrylat, Cerotinyl(meth)acrylat, Melissinyl(meth)acrylat, Palmitoleinyl(meth)acrylat, Oleyl(meth)acrylat, Linolyl(meth)acrylat, Linolenyl(meth)acrylat, Stearyl(meth)acrylat, Lauryl(meth)acrylat, n-Octyl(meth)acrylamid, n-Nonyl(meth)acrylamid, n-Decyl(meth)acrylamid, n-Undecyl(meth)acrylamid, Tridecyl(meth)acrylamid, Myristyl(meth)acrylamid, Pentadecyl(meth)acrylamid, Palmityl(meth)acrylamid, Heptadecyl(meth)acrylamid, Nonadecyl(meth)acrylamid, Arachinyl(meth)acrylamid, Behenyl(meth)acrylamid, Lignocerenyl(meth)acrylamid, Cerotinyl(meth)acrylamid, Melissinyl(meth)acrylamid, Palmitoleinyl(meth)acrylamid, Oleyl(meth)acrylamid, Linolyl(meth)acrylamid, Linolenyl(meth)acrylamid, Stearyl(meth)acrylamid, Lauryl(meth)acrylamid und Mischungen davon.

Bevorzugt als Komponente d) sind weiterhin Verbindungen der allgemeinen Formel II, worin
R³ für Wasserstoff, Methyl oder Ethyl steht,
X² für O oder NH steht, und
R⁴ für Wasserstoff steht, und im Falle von X² = O auch deren Salze.

Bevorzugte Verbindungen d) sind primäre Amide α,β-ethylenisch ungesättigter Monocarbonsäuren, wie Acrylamid, Methacrylamid, Ethacrylamid etc.

Besonders bevorzugte Verbindungen d) sind Verbindungen der Formel II mit X² = O und R⁴ = H, wie Acrylsäure, Methacrylsäure, Ethacrylsäure, sowie deren Alkalimetallsalze, z. B. deren Natrium- und Kaliumsalze, und Mischungen davon. Beim Einsatz dieser säuregruppenhaltigen Verbindungen d) resultieren Polymere mit anionogenen Gruppen, die durch Neutralisation, wie im Folgenden beschrieben, teilweise oder vollständig in anionische Gruppen überführt werden können. Selbstverständlich können auch die Monomere d) bereits in Salzform vorliegen.

Die zuvor genannten Verbindungen der Komponente d) können einzeln oder in Form von Mischungen eingesetzt werden.

In einer bevorzugten Ausführungsform umfasst die Komponente d) wenigstens eine Verbindung der allgemeinen Formel II mit X² = O und R⁴ = H in einer Menge von 0,1 bis 20 und vorzugsweise in einer Menge von 0,2 bis 10 Gew.-% bezogen auf die Gesamtmenge der Monomere a), b), c) und d). Hierunter bevorzugt sind die Monomere Acrylsäure und Methacrylsäure. Selbstverständlich kann die Komponente d) neben den Monomeren der Formel II mit X² = O und R⁴ = H auch davon verschiedene Monomere umfassen (R⁴ ≠ H, wenn X² = O).

Die in den erfindungsgemäßen Mitteln eingesetzten Polymere können wenigstens eine weitere Komponente, die ausgewählt ist unter
e) von d) verschiedenen Verbindungen mit einer radikalisch polymerisierbaren α,β-ethylenisch ungesättigten Doppelbindung und mindestens einer anionogenen und/oder anionischen Gruppe pro Molekül,
f) vernetzend wirkenden Monomeren mit mindestens zwei ethylenisch ungesättigten, nicht konjugierten Doppelbindungen,
g) von a) bis f) verschiedenen radikalisch polymerisierbaren Monomeren,
und Mischungen davon, einpolymerisiert enthalten.

Die in den erfindungsgemäßen kosmetischen Mitteln eingesetzten wasserlöslichen oder wasserdispergierbaren Polymere können zusätzlich zu den zuvor genannten Monomerkomponenten noch eine Komponente e) einpolymerisiert enthalten, die ausgewählt ist unter von d) verschiedenen Verbindungen mit einer radikalisch polymerisierbaren, α,β-ethylenisch ungesättigten Doppelbindung und mindestens einer anionogenen und/oder anionischen Gruppe pro Molekül und Mischungen davon.

Vorzugsweise sind die anionogenen und anionischen Gruppen der Verbindungen der Komponente e) ausgewählt unter Carboxylat- und/oder Sulfonatgruppen und deren durch teilweise oder vollständige Neutralisation mit einer Base erhältlichen Salze.

Geeignete Verbindungen e) sind z. B. ethylenisch ungesättigte Mono- und/oder Dicarbonsäuren und deren Halbester und Anhydride, wie Fumarsäure, Maleinsäure, Itaconsäure, Crotonsäure, Maleinsäureanhydrid, Monobutylmaleat etc. und deren Alkalimetallsalze, wie deren Natrium- und Kaliumsalze.

Geeignete Monomere e) sind weiterhin Acrylamidoalkansulfonsäuren und deren Salze, wie 2-Acrylamido-2-methylpropansulfonsäure und deren Alkalimetallsalze, z. B. deren Natrium- und Kaliumsalze.

Die wasserlöslichen oder wasserdispergierbaren Polymere enthalten die verbindungen der Komponenten d) und e) mit wenigstens einer anionogenen und/oder anionischen Gruppe pro Molekül vorzugsweise in einer Menge von 0 bis 15 Gew.-%, bevorzugt 0,1 bis 12 Gew.-%, bezogen auf die Gesamtmenge der zu polymerisierenden Monomere, einpolymerisiert.

Die in den erfindungsgemäßen Mitteln eingesetzten Polymere können noch wenigstens ein von den Verbindungen der Komponenten a) bis e) verschiedenes vernetzend wirkendes Monomer f) einpolymerisiert enthalten, das wenigsterns zwei α,β-ethylenisch ungesättigte Doppelbindungen pro Molekül aufweist.

Die erfindungsgemäßen Mittel enthalten die Komponente f) vorzugsweise in einer Menge von 0,001 bis 4 Gew.-%, insbesondere 0,01 bis 2 Gew.-%, bezogen auf die Gesamtmenge aller das Polymer bildenden Komponenten.

Geeignete Vernetzer f) sind zum Beispiel Acrylester, Methacrylester, Allylether oder Vinylether von mindestens zweiwertigen Alkoholen. Die OH-Gruppen der zugrundeliegenden Alkohole können dabei ganz oder teilweise verethert oder verestert sein; die Vernetzer enthalten aber mindestens zwei ethylenisch ungesättigte Gruppen.

Beispiele für die zugrundeliegenden Alkohole sind zweiwertige Alkohole, wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglycolmonoester, 2,2-Bis(4-hydroxyphenyl)propan, 2,2-Bis[4-(2-hydroxypropyl)phenyl]propan, Diethylenglykol, Tri-ethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10 000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Tri-ethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epi-chlorhydrin in die entsprechenden Glycidylether überführt werden.

Weitere geeignete Vernetzer f) sind die Vinylester oder die Ester einwertiger, ungesättigter Alkohole mit ethylenisch ungesättigten C₃- bis C₆-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellitsäure, Phthalsäure, Terephthalsäure, Citronensäure oder Bernsteinsäure.

Weitere geeignete Vernetzer sind Ester ungesättigter Carbonsäuren mit den oben beschriebenen mehrwertigen Alkoholen, beispielsweise der Ölsäure, Crotonsäure, Zimtsäure oder 10-Undecensäure.

Geeignet als Monomere (f) sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z.B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20 000.

Als Vernetzer sind ferner geeignet die Acrylsäureamide, Meth-acrylsäureamide und N-Allylamine von mindestens zweiwertigen Aminen. Solche Amine sind zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylmonoalkylammoniumsalze, z.B. Triallylmethylammoniumchlorid oder -methylsulfat, als Vernetzer geeignet.

Geeignete Vernetzter sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z.B. N,N'-Divinylethylenharnstoff oder N,N'-Divinylpropylenharnstoff.

Geeignete Vernetzer sind auch die in der DE-A-19838852 genannten Urethanacrylate.

Weitere geeignete Vernetzer sind Divinyldioxan, Tetraallylsilan oder Tetravinylsilan.

Vorzugsweise werden solche Vernetzer eingesetzt, die in der Monomermischung löslich sind.

Bevorzugt eingesetzte Vernetzer sind beispielsweise Pentaerythrittriallylether, Methylenbisacrylamid, Triallylamin und Triallylalkylammoniumsalze, Divinylimidazol, N,N'-Divinyl-ethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind.

Die Monomere f) können jeweils einzeln oder im Gemisch mit anderen Monomeren der gleichen Gruppe eingesetzt werden.

Der Einsatz wenigstens einer vernetzenden Verbindung f) führt in der Regel zu Polymeren mit einem höheren Molekulargewicht als entsprechende in Abwesenheit der Komponente f) erhaltene Polymere. Polymere, die wenigstens eine Verbindung der Komponente f) einpolymerisiert enthalten, eignen sich vorzugsweise für kosmetische Mittel in Form eines Shampoos, Haarschaums oder Haargels.

Die in den erfindungsgemäßen Mitteln eingesetzten Polymere können noch wenigstens ein weiteres von den Verbindungen der Komponenten a) bis f) verschiedenes radikalisch polymerisierbares Monomer g) einpolymerisiert enthalten.

Die erfindungsgemäßen Mittel enthalten die Komponente g) vorzugsweise in einer Menge von 0,1 bis 10 Gew.-%, insbesondere 0,5 bis 5 Gew.-%, bezogen auf die Gesamtmenge aller das Polymer bildenden Komponenten.

Geeignete Monomere g) sind z. B. Vinylaromaten, wie Styrol, α-Methylstyrol, o-Chlorstyrol und Vinyltoluole, Vinylhalogenide, wie Vinylchlorid, Vinylidenhalogenide, wie Vinylidenchlorid, Monoolefine, wie Ethylen und Propylen, nichtaromatische Kohlenwasserstoffe mit mindestens 2 konjugierten Doppelbindungen, wie Butadien, Isopren und Chloropren, sowie Mischungen davon.

Vorzugsweise enthält das Polymer
- 7 bis weniger als 50 Gew.-%, bevorzugt 10 bis 45 Gew.-%, wenigstens einer Komponente a),
- 30 bis 87 Gew.-%, bevorzugt 40 bis 85 Gew.-%, insbesondere 50 bis 80 Gew.-%, wenigstens einer Komponente b),
- 1 bis 25 Gew.-%, bevorzugt 3 bis 20 Gew.-%, wenigstens einer Komponente c),
- 0 bis 25 Gew.-%, bevorzugt 0,1 bis 20 Gew.-% wenigstens einer Komponente d), wobei die Komponente d) insbesondere 1 bis 10 Gew.-% einer Verbindung II mit X² = O und R⁴ = H umfasst, und, sofern erwünscht,
- 0 bis 15 Gew.-%, bevorzugt 0,1 bis 12 Gew.-%, wenigstens einer Komponente e),
einpolymerisiert.

Bevorzugt werden bei der Herstellung der erfindungsgemäßen, wasserlöslichen bzw. wasserdispergierbaren Polymere die Komponenten c), d) und gegebenenfalls e) in solchen Mengen eingesetzt, dass das Verhältnis von Äquivalenten kationogener Gruppen größer oder gleich den Äquivalenten anionogener Gruppen ist.

Bevorzugt sind Polymere, die
- wenigstens eine Verbindung der Komponente a), ausgewählt unter tert.-Butylacrylat, tert.-Butylmethacrylat, N-tert.-Butylacrylamid, N-tert.-Butylmethacrylamid und Mischungen davon,
- wenigstens eine Verbindung der Komponente b), ausgewählt unter N-Vinylformamid, N-Vinylpyrrolidon, N-Vinylcaprolactam und Mischungen davon,
- wenigstens eine Verbindung der Komponente c), ausgewählt unter Dimethylaminoethylacrylat,
   Dimethylaminoethylmethacrylat (DMAEMA),
   N-[3-(dimethylamino)propyl]acrylamid,
   N-[3-(dimethylamino)propyl]methacrylamid (DMAPMA) und Mischungen davon,
- Acrylsäure und/oder Acrylamid und/oder Methacrylsäure als Verbindung der Komponente d) und gegebenenfalls eine oder mehrere weitere Verbindungen der Komponente d), ausgewählt unter n-Butylacrylat, n-Butylmethacrylat, n-Stearylacrylat, n-Stearylmethacrylat, n-Laurylacrylat, n-Laurylmethacrylat,
einpolymerisiert enthalten.

Besonders bevorzugt sind Polymere, die N-[3-(dimethylamino)propyl]methacrylamid und (Meth)acrylsäure im Gewichtsmengenverhältnis 2:0,9 bis 2:1,1 einpolymerisiert enthalten.

Nach einer bevorzugten Ausführungsform enthalten die erfindungsgemäßen kosmetischen Mittel zusätzlich wenigstens ein Polyalkylenoxidgruppen-haltiges Polysiloxan. Die Mittel können diese Siloxane in Form einer separaten Komponente und/oder eingebaut in eines der zuvor beschriebenen wasserlöslichen oder wasserdispergierbaren Polymere enthalten. Der Einbau der Polysiloxane erfolgt vorzugweise durch Pfropfung, d. h. Polymerisation der Monomere a) bis d) und gegebenenfalls e) bis g) in Gegenwart der Polysiloxane.

In einer bevorzugten Ausführungsform der Erfindung enthalten die wasserlöslichen oder wasserdispergierbaren Polymere ein Polyalkylenoxidgruppen-haltiges Polysiloxan eingebaut. Die Menge an Polysiloxan beträgt dann vorzugsweise 1 bis 20 Gew.-%, bezogen auf die Gesamtmenge an Monomeren a), b), c), d), gegebenenfalls e) und Polysiloxan. In dieser Ausführungsform umfassen die Monomere d) vorzugsweise wenigstens eine Verbindung der Formel II mit X² = R und R⁴ = H, insbesondere Acrylsäure und/oder Methacrylsäure oder deren Salze in einer Menge von 1 bis 10 Gew.-%, bezogen auf die Gesamtmenge der Komponenten a), b), c), d), gegebenenfalls e) und Polysiloxan, sowie gegebenenfalls weitere Verbindungen der Formel II.

Vorzugsweise ist das Polysiloxan ausgewählt unter Verbindungen der allgemeinen Formel III 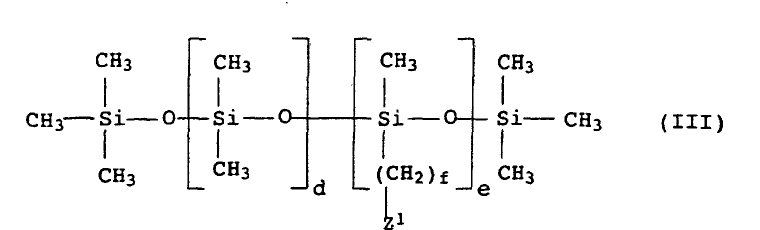 worin
die Reihenfolge der Siloxaneinheiten beliebig ist,
- d und e: unabhängig voneinander für 0 bis 100 stehen, wobei die Summe aus d und e mindestens 2 ist,
- f: für eine ganze Zahl von 2 bis 8 steht,
- Z¹: für einen Rest der Formel IV

-(CH₂)ᵤ-O-(CH₂CH₂O)ᵥ(CH₂CH(CH₃)O)_{w}-R^{A} (IV)

steht, wobei
in der Formel IV die Reihenfolge der Alkylenoxideinheiten beliebig ist,
u für eine ganze Zahl von 1 bis 8 steht,
v und w unabhängig voneinander für eine ganze Zahl von 0 bis 200 stehen, wobei die Summe aus v und w > 0 ist, und
R^{A} = H oder C₁-C₆-Alkyl, insbesondere H, CH₃ oder C₂H₅ ist.

Bevorzugt weisen die Polysiloxane ein Molekulargewicht in einem Bereich von etwa 300 bis 30000 auf.

Bevorzugt liegt die Gesamtzahl der Alkylenoxideinheiten der Polysiloxane, d. h. die Summe aus v und w in der Formel IV, in einem Bereich von etwa 3 bis 200, bevorzugt 5 bis 180.

Geeignete Polysiloxane sind die unter dem internationalen Freinamen Dimethicone Copolyole oder als Silicontenside bekannten Verbindungen, wie z. B. die unter den Markennamen Belsil® (Fa. Wacker) oder Silvet® (Fa. Witco) erhältlichen Verbindungen. Bevorzugt ist z. B. Belsil@ 6031 oder Silvet®L.

Vorzugsweise liegt das Gewichtsmengenverhältnis von wasserlöslichem oder wasserdispergierbarem Polymer zu Polysiloxan in einem Bereich von etwa 70:30 bis 99,9:0,1, bevorzugt etwa 85:15 bis 99:1.

Ein weiterer Gegenstand der Erfindung ist ein wasserlösliches oder wasserdispergierbares Polymer, das
a) 5 bis weniger als 50 Gew.-% wenigstens eines α,β-ethylenisch ungesättigten Monomers der allgemeinen Formel I 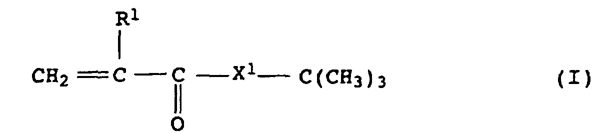 worin
   - R¹: für Wasserstoff oder C₁- bis C₈-Alkyl steht, und
   - X¹: für O oder NR² steht, wobei R² für Wasserstoff, C₁- bis C₈-Alkyl oder C₅- bis C₈-Cycloalkyl steht,
b) 25 bis 90 Gew.-% wenigstens eines N-Vinylamids und/oder N-Vinyllactams,
c) 0,5 bis 30 Gew.-% wenigstens einer Verbindung mit einer radikalisch polymerisierbaren, α,β-ethylenisch ungesättigten Doppelbindung und mindestens einer kationogenen und/oder kationischen Gruppe pro Molekül,
d) 0,1 bis 30 Gew.-% wenigstens eines α,β-ethylenisch ungesättigten Monomers der allgemeinen Formel II 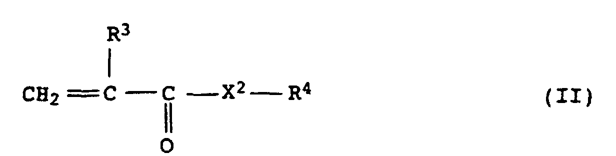 worin
   - R³: für Wasserstoff oder C₁- bis C₈-Alkyl steht,
   - X²: für O oder NR⁵ steht, wobei R⁵ für Wasserstoff, C₁- bis C₈-Alkyl oder C₅- bis C₈-Cycloalkyl steht, und
   - R⁴: für Wasserstoff oder einen linearen C₁- bis C₂₂-Alkylrest steht,
   einpolymerisiert enthält. Hinsichtlich der bevorzugten Monomere a), b), c) und d) gilt das zuvor Gesagte ebenso wie für die Mengenanteile der Monomere a), b), c) und d) bezogen auf die Gesamtmonomermenge.

Die erfindungsgemäßen Polymere können noch wenigstens eine weitere Komponente, die ausgewählt ist unter
e) von d) verschiedenen Verbindungen mit einer radikalisch polymerisierbaren α,β-ethylenisch ungesättigten Doppelbindung und mindestens einer anionogenen und/oder anionischen Gruppe pro Molekül,
f) vernetzend wirkenden Monomeren mit mindestens zwei ethylenisch ungesättigten, nicht konjugierten Doppelbindungen,
g) von a) bis f) verschiedenen radikalisch polymerisierbaren Monomeren,
und Mischungen davon, einpolymerisiert enthalten.

Besonders bevorzugt umfasst die Komponente d) wenigstens eine Verbindung II mit X² = O und R⁴ = H, insbesondere Acrylsäure und/oder Methacrylsäure, in einer Menge von 0,1 bis 20 Gew.-% und insbesondere 1 bis 10 Gew.-%, bezogen auf die Gesamtmenge der Monomere a), b), c), d) und gegebenenfalls weiteren Komponenten. Daneben kann die Komponente d) auch davon verschiedene Verbindungen der Formel II (d. h. R⁴ ≠ H, wenn X² = O) umfassen, z. B. in einer Menge von 0,1 bis 25 Gew.-%. Selbstverständlich können die erfindungsgemäßen Polymere auch die vorstehend genannten Monomere der Komponente e) einpolymerisiert enthalten.

In einer bevorzugten Ausführungsform enthalten diese Polymere wenigstens eines der oben definierten Polysiloxane eingebaut, insbesondere eines der allgemeinen Formel III. Die Menge an Polysiloxan beträgt dann vorzugsweise 1 bis 20 Gew.-% bezogen auf die Gesamtmenge an Monomeren a), b), c) und d), gegebenenfalls e) und Polysiloxan. Derartige Polymere enthalten in der Regel 1 bis 10 Gew.-% wenigstens eines Monomers d) der Formel II mit X² = O und R⁴ = H und gegebenenfalls zusätzlich weitere Monomere der Formel II einpolymerisiert, wobei die Menge ebenfalls auf die Gesamtmenge von a), b), c), d), gegebenenfalls e) und Polysiloxan bezogen ist.

Besonders bevorzugt enthalten derartige Polymere einpolymerisiert:
- 10 bis weniger als 50 Gew.-%: wenigstens eines Monomers der Formel I (Komponente a)),
- 25 bis 70 Gew.-%: wenigstens eines N-Vinylamids und/oder N-Vinyllactams (Komponente b)),
- 3 bis 20 Gew.-%: wenigstens eines der unter c) genannten Monomere,
- 1 bis 10 Gew.-%: wenigstens eines Monomers der Formel II mit X² = O und R⁴ = H,
- 0 bis 25 Gew.-%: eines oder mehrere Monomere der Formel II mit R⁴ ≠ H, wenn X² = 0 und
- 1 bis 20 Gew.-%: wenigstens eines Polysiloxans mit Polyalkylenoxidgruppen, insbesondere ein Polysiloxan der Formel II, wobei alle angegebenen Gewichtsanteile sich zu 100 Gew.-% addieren.

Die Herstellung der in den erfindungsgemäßen Mitteln eingesetzten Polymere sowie der erfindungsgemäßen Polymere erfolgt durch radikalische Polymerisation nach üblichen, dem Fachmann bekannten Verfahren. Dazu zählt die radikalische Polymerisation in Masse, Emulsion, Suspension und in Lösung, vorzugsweise die Emulsions- und Lösungspolymerisation. Die Mengen an zu polymerisierenden Verbindungen, bezogen auf Lösungs- bzw. Dispergiermittel, werden dabei im Allgemeinen so gewählt, dass etwa 30 bis 80 gew.-%ige Lösungen, Emulsionen oder Dispersionen erhalten werden. Die Polymerisationstemperatur beträgt in der Regel 30 bis 120 °C, bevorzugt 40 bis 100 °C.

Das Polymerisationsmedium für die Lösungspolymerisation kann sowohl nur aus einem organischen Lösungsmittel als auch aus Mischungen aus Wasser und mindestens einem wassermischbaren, organischen Lösungsmittel bestehen. Bevorzugte organische Lösungsmittel sind z. B. Alkohole, wie Methanol, Ethanol, n-Propanol, Isopropanol, n-Butanol, Ketone, wie Aceton und Methylethylketon, Tetrahydrofuran etc. Werden zur Polymerisation Monomere d) und/oder e) mit anionogenen und/oder anionischen Gruppen, wie z. B. Acrylsäure, Methacrylsäure etc., eingesetzt, so erfolgt die Polymerisation vorzugsweise in einem Gemisch aus Wasser und mindestens einem wassermischbaren organischen Lösungsmittel.

Werden zur Polymerisation Monomere eingesetzt, wobei die Komponente b) keine N-Vinylamide umfasst, so kann der pH-Wert bei der Polymerisation im Allgemeinen in einem weiten Bereich von schwach sauer über neutral bis alkalisch variiert werden. wird zur Polymerisation wenigstens ein N-Vinylamid b) eingesetzt, so liegt der pH-Wert des Reaktionsgemisches, beginnend mit der Zugabe dieses Monomers, vorzugsweise in einem Bereich von 6 bis 8, insbesondere von 6,2 bis 7,2.

Die Lösungspolymerisation kann sowohl als Batchprozess als auch in Form eines Zulaufverfahrens, einschließlich Monomerenzulauf, Stufen- und Gradientenfahrweise, durchgeführt werden. Bevorzugt ist im Allgemeinen das Zulaufverfahren, bei dem man gegebenenfalls einen Teil des Polymerisationsansatzes vorlegt, auf die Polymerisationstemperatur erhitzt und anschließend den Rest des Polymerisationsansatzes, üblicherweise über einen oder auch mehrere, räumliche getrennte Zuläufe, kontinuierlich, stufenweise oder unter Überlagerung eines Konzentrationsgefälles unter Aufrechterhaltung der Polymerisation der Polymerisationszone zuführt.

Werden zur Polymerisation Monomere d) und/oder e) mit anionogenen und/oder anionischen Gruppen eingesetzt, so kann deren Zugabe gemeinsam mit den Monomeren mit kationogene und/oder kationischen Gruppen c) oder zeitlich getrennt von diesen erfolgen. Bevorzugt erfolgt die Zugabe der Monomere mit anionogenen und/oder anionischen Gruppen nach Abschluss der Zugabe der Monomere mit kationogenen und/oder kationischen Gruppen. Gewünschtenfalls können die Monomere mit anionogenen Gruppen, wie z. B. Acrylsäure, Methacrylsäure etc., auch als Neutralisationsmittel nach Beendigung der Polymerisation zugegeben werden.

Als Initiatoren für die radikalische Polymerisation werden übliche Peroxo- oder Azoverbindungen eingesetzt. Dazu zählen z.B. Dibenzoylperoxid, tert.-Butylperpivalat, tert.-Butylper-2-ethylhexanoat, Di-tert.-butylperoxid, 2,5-Dimethyl-2,5-di(tert.-butylperoxy)hexan, aliphatische oder cycloaliphatische Azoverbindungen, z. B. 2,2'-Azobis(isobutyronitril), 2,2'-Azobis(2-methylbutyronitril), 2,2'-Azobis(2,4-dimethylvaleronitril), 1,1'-Azobis(1-cyclohexancarbonitril), 2-(Carbamoylazo)isobutyronitril, 4,4'-Azobis(4-cyanovaleriansäure) und deren Alkalimetall- und Ammoniumsalze, z. B. das Natriumsalz, Dimethyl-2,2'-azobisisobutyrat, 2,2'-Azobis[2-(2-imidazolin-2-yl)propan], 2,2'-Azobis(2-amidinopropan) und die Säureadditionssalze der beiden zuletzt genannten Verbindungen, z. B. die Dihydrochloride.

Ferner kommen als Initiatoren Wasserstoffperoxid, Hydroperoxide in Kombination mit Reduktionsmitteln und Persalze in Frage. Geeignete Hydroperoxide sind beispielsweise t-Butylhydroperoxid, t-Amylhydroperoxid, Cumolhydroperoxid und Pinanhydroperoxid jeweils in Kombination mit beispielsweise einem Salz der Hydroxymethansulfinsäure, einem Eisen (II)-Salz oder Ascorbinsäure. Geeignete Persalze sind insbesondere Alkalimetallperoxidisulfate.

Die verwendete Initiatormenge, bezogen auf die Monomere, liegt im Allgemeinen in einem Bereich von etwa 0,1 bis 2 Gew.-% bezogen auf das Gesamtgewicht der zu polymerisierenden Monomere.

Zur Erzielung eines gewünschten K-Wertes der Polymere kann, insbesondere bei der Emulsions- oder Suspensionspolymerisation, der Einsatz eines Reglers angebracht sein. Als Regler eignen sich beispielsweise Aldehyde, wie Formaldehyd, Acetaldehyd, Propionaldehyd, n-Butyraldehyd und Isobutyraldehyd, Ameisensäure, Ammoniumformiat, Hydroxylammoniumsulfat und Hydroxylammoniumphosphat. Weiterhin können Regler eingesetzt werden, die Schwefel in organisch gebundener Form enthalten, wie Di-n-butylsulfid, Di-n-octylsulfid, Diphenylsulfid etc., oder Regler, die Schwefel in Form von SH-Gruppen enthalten, wie n-Butylmercaptan, n-Hexylmercaptan oder n-Dodecylmercaptan. Geeignet sind auch wasserlösliche, schwefelhaltige Polymerisationsregler, wie beispielsweise Hydrogensulfite und Disulfite. Weiterhin eignen sich als Regler Allylverbindungen, wie Allylalkohol oder Allylbromid, Benzylverbindungen, wie Benzylchlorid oder Alkylhalogenide, wie Chloroform oder Tetrachlormethan.

Gewünschtenfalls setzt man der Polymerlösung im Anschluss an die Polymerisationsreaktion einen oder mehrere Polymerisationsinitiatoren zu und erhitzt die Polymerlösung, z. B. auf die Polymerisationstemperatur oder auf Temperaturen oberhalb der Polymerisationstemperatur, um die Polymerisation zu vervollständigen. Geeignet sind die oben angegebenen Azoinitiatoren, aber auch alle anderen üblichen, für eine radikalische Polymerisation in wässriger Lösung geeignete Initiatoren, beispielsweise Peroxide, Hydroperoxide, Peroxodisulfate, Percarbonate, Peroxoester und Wasserstoffperoxid. Hierdurch wird die Polymerisationsreaktion zu einem höheren Umsatz, wie z. B. von 99,9 %, geführt. Eine Reduzierung des Restmonomergehalts gelingt alternativ auch durch eine saure Hydrolyse. Hierzu werden die nach dem oben beschriebenen Verfahren erhaltenen Polymerlösungen mit Wasser und einer Säure, beispielsweise einer organischen Säure wie Milchsäure, oder einer Mineralsäure wie Schwefelsäure, Salzsäure oder Phosphorsäure, versetzt und erwärmt, vorzugsweise auf Temperaturen ≤ 100 °C, z. B. 50 bis 100 °C. Die Menge an Säure wird hierzu vorzugsweise so gewählt, dass ein pH-Wert im Bereich von 4,5 bis 5,5 erreicht wird. Das Erwärmen wird vorzugsweise als Wasserdampfdestillation ausgestaltet, wobei wasserdampfflüchtige Restmonomere oder deren Hydrolyseprodukte sowie gegebenenfalls flüchtige Lösungsmittel entfernt werden. Vorzugsweise führt man eine Wasserdampfdestillation durch, bis eine Kopftemperatur von etwa 100 °C erreicht ist.

Die bei der Polymerisation entstehenden Lösungen können gegebenenfalls durch ein dem Stand der Technik entsprechendes Trocknungsverfahren in feste Pulver überführt werden. Bevorzugte Verfahren sind beispielsweise die Sprühtrocknung, die Sprühwirbelschichttrocknung, die Walzentrocknung und die Bandtrocknung. Ebenfalls anwendbar sind die Gefriertrocknung und die Gefrierkonzentrierung. Gewünschtenfalls kann das Lösungsmittel auch durch übliche Methoden, z. B. Destillation bei verringertem Druck, teilweise oder vollständig entfernt werden.

Nach einer bevorzugten Ausführungsform wird als wasserlösliches oder wasserdispergierbares Polymer ein Polymer eingesetzt, das durch radikalische Polymerisation der das Polymer bildenden Monomere in Gegenwart von wenigstens einem der Polyalkylenoxidgruppen-haltigen Polysiloxane erhalten wurde.

Die Polymerisation in Gegenwart wenigstens eines Polysiloxans erfolgt nach den zuvor beschriebenen Verfahren, wobei die Polysiloxankomponente als Bestandteil der Vorlage, eines Monomerzulaufs oder als separater Zulauf eingesetzt werden kann.

Bei den in den erfindungsgemäßen Mitteln eingesetzten wasserlöslichen oder wasserdispergierbaren Polymeren handelt es sich um kationische bzw. kationogene Polymere. Geladene kationische Gruppen lassen sich aus den Aminstickstoffen der kationogenen Gruppen entweder durch Protonierung, z. B. mit Carbonsäuren, wie Milchsäure, oder Mineralsäuren, wie Phosphorsäure, Schwefelsäure und Salzsäure oder durch Quaternisierung, z. B. mit Alkylierungsmitteln, wie C₁- bis C₄-Alkylhalogeniden oder -sulfaten erzeugen. Dies kann sowohl vor der Polymerisation durch entsprechende Umsetzung der Monomere als auch nach der Polymerisation erfolgen. Die Neutralisation kann je nach Anwendungszweck partiell, z. B. zu 5 bis 95 %, vorzugsweise 30 bis 95 %, oder vollständig, d. h. zu 100 % erfolgen.

Polymere, die zusätzlich wenigstens eine Verbindung der Komponenten d) und/oder e) mit anionogenen Gruppen einpolymerisiert enthalten, können mit einer Base teilweise oder vollständig neutralisiert werden. Als Base für die Neutralisation der Polymere können Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxyd, Calciumoxid, Magnesiumhydroxyd oder Magnesiumcarbonat sowie Ammoniak und Amine verwendet werden. Geeignete Amine sind z. B. C₁-C₆-Alkylamine, bevorzugt n-Propylamin und n-Butylamin, Dialkylamine, bevorzugt Diethylpropylamin und Dipropylmethylamin, Trialkylamine, bevorzugt Triethylamin und Triisopropylamin, C₁-C₆-Alkyldiethyanolamine, bevorzugt Methyl- oder Ethyldiethanolamin und Di-C₁-C₆-Alkylethanolamine sowie Glucamin und Methylglucamin. Besonders für den Einsatz in Haarbehandlungsmitteln haben sich zur Neutralisation der Säuregruppen enthaltenden Polymere 2-Amino-2-methyl-1-propanol, Diethylaminopropylamin und Triisopropanolamin bewährt. Die Neutralisation der Säuregruppen enthaltenden Polymere kann auch mit Hilfe von Mischungen mehrerer Basen vorgenommen werden, z. B. Mischungen aus Natronlauge und Triisopropanolamin. Die Neutralisation kann je nach Anwendungszweck partiell z. B. zu 20 bis 40 % oder vollständig, d. h. zu 100 % erfolgen.

Polymere mit ionogenen und ionischen Gruppen stellen gegebenenfalls amphotere Systeme dar, deren Eigenschaften in Abhängigkeit vom pH-Wert variiert werden können. Zur Einstellung des pH-Wertes von Formulierungen auf Basis dieser Polymere können die zuvor genannten Säuren und Basen eingesetzt werden. In aller Regel weisen die erhaltenen Salze der Polymere eine bessere Wasserlöslichkeit oder Dispergierbarkeit in Wasser auf als die nicht neutralisierten Polymere.

Polymere, die sowohl kationogene als auch anionogene Gruppen aufweisen, können nacheinander einer Neutralisation mit wenigstens einer Säure, einer Neutralisation mit wenigstens einer Base und gewünschtenfalls zusätzlich einer Quaternisierung unterzogen werden. Die Reihenfolge der Neutralisationsschritte ist dabei im Allgemeinen beliebig. Wasserlösliche oder wasserdispergierbare Polymere mit freien kationischen (Amin-)Gruppen oder mit freien anionischen (Säure-)Gruppen können als "makromolekulares Neutralisationsmittel" mit wenigstens einem weiteren ionogenen Polymer zu einem wasserdispergierbaren oder wasserlöslichen polymeren Säure-Base-Komplex umgesetzt werden. Diese eignen sich für vielfältige kosmetische Anwendungen. Geeignete Polymere werden im Folgenden beschrieben.

Die in den erfindungsgemäßen Mitteln eingesetzten Polymere weisen K-Werte (gemessen nach E. Fikentscher, Cellulose-Chemie 13 (1932), Seite 58-64, an einer 1 gew.-%igen Lösung in Ethanol) in einem Bereich von etwa 15 bis 90, bevorzugt 20 bis 60, auf. Ihre Glasübergangstemperatur beträgt im Allgemeinen mindestens 0 °C, bevorzugt mindestens 20 °C, insbesondere bevorzugt mindestens 25 °C. Üblicherweise liegt die Glasübergangstemperatur dann in einem Bereich von etwa 30 bis 130 °C, insbesondere 40 bis 100 °C.

Die in den erfindungsgemäßen Mitteln enthaltenen Polymere sind als Hilfsmittel in der Kosmetik und Pharmazie, insbesondere als oder in Beschichtungsmittel(n) für keratinhaltige Oberflächen (Haar, Haut und Nägel) und als Überzugsmittel und/oder Bindemittel für feste Arzneiformen brauchbar. Außerdem sind sie als oder in Beschichtungsmittel(n) für die Textil-, Papier-, Druck-, Leder- und Klebstoffindustrie brauchbar. Sie sind insbesondere in der Haarkosmetik brauchbar. Die zuvor genannten Polymere können auch in Cremes und als Tablettenüberzugmittel und Tablettenbindemittel verwendet werden. Sie eignen sich auch als Bindemittel und Klebemittel für kosmetische Produkte, z. B. bei der Herstellung von Schminken, wie Mascara und Rouge und bei der Herstellung stiftförmiger, kosmetischer Produkte, wie Deostifte, Schminkstifte etc.

Die erfindungsgemäßen kosmetischen Mittel eignen sich insbesondere als Beschichtungsmittel für keratinhaltige Oberflächen (Haar, Haut und Nägel). Speziell eignen sich die erfindungsgemäßen und erfindungsgemäß eingesetzten Polymere als Hilfs- oder Wirkstoffe in haarkosmetischen Mitteln. Dazu zählen die Verwendung als Festigerpolymer in Haarsprays und Schaumfestigern, als Conditioner und Verdicker in Haarmousse, Haargel und Shampoos. Bevorzugt sind Haargele, die neben den erfindungsgemäßen und erfindungsgemäß eingesetzten Polymeren keine weiteren Verdicker enthalten. Die in ihnen eingesetzten Verbindungen sind wasserlöslich oder wasserdispergierbar. Sind die in den erfindungsgemäßen Mitteln eingesetzten Verbindungen wasserdispergierbar, können sie in Form von wässrigen Mikrodispersionen mit Teilchendurchmessern von üblicherweise bis 500 nm, bevorzugt 1 bis 300 nm, zur Anwendung gebracht werden. Die Feststoffgehalte der Präparate liegen dabei üblicherweise in einem Bereich von etwa 0,2 bis 20 Gew.-%, bevorzugt 0,5 bis 12 Gew.-%. Diese Mikrodispersionen benötigen in der Regel keine Emulgatoren oder Tenside zu ihrer Stabilisierung.

Bevorzugt können die erfindungsgemäßen Mittel in Form eines Haarbehandlungsmittels, insbesondere in Form eines Haarsprays vorliegen. Zur Anwendung als Haarfestiger sind dabei Mittel bevorzugt, die Polymere enthalten, die wenigstens eine Glasübergangstemperatur T_{g} ≥ 20 °C, bevorzugt ≥ 30 °C, aufweisen. Der K-Wert dieser Polymere liegt vorzugsweise in einem Bereich von 23 bis 90, insbesondere 25 bis 60.

Im Allgemeinen enthalten die erfindungsgemäßen kosmetischen Mittel die Polymere in einer Menge im Bereich von 0,2 bis 20 Gew.-%, bezogen auf das Gesamtgewicht des Mittels.

Vorzugsweise handelt es sich um Haarbehandlungsmittel. Diese liegen üblicherweise in Form einer wässrigen Dispersion oder in Form einer alkoholischen oder wässrig-alkoholischen Lösung vor. Beispiele geeigneter Alkohole sind Ethanol, Propanol, Isopropanol etc.

Weiter enthalten die erfindungsgemäßen Haarbehandlungsmittel im Allgemeinen übliche kosmetische Hilfsstoffe, beispielsweise Weichmacher, wie Glycerin und Glykol; Emollienzien; Parfüms; UV-Absorber; Farbstoffe; antistatische Mittel; Mittel zur Verbesserung der Kämmbarkeit; Konservierungsmittel; Wachse, insbesondere Fettamide; und Entschäumer.

Wenn die erfindungsgemäßen Mittel als Haarspray formuliert sind, enthalten sie eine ausreichende Menge eines Treibmittels. Als Treibmittel werden vorzugsweise Kohlenwasserstoffe (LPG), insbesondere Propan, n-Butan, n-Pentan und Gemische davon eingesetzt. Geeignete niedrigsiedende Treibmittel sind weiterhin Ether, bevorzugt Dimethylether. Gewünschtenfalls können als Treibmittel aber auch komprimierte Gase, wie Stickstoff, Luft oder Kohlendioxid, eingesetzt werden. Die in den erfindungsgemäßen Mitteln eingesetzten, zuvor genannten Polymere weisen eine hohe Treibgasverträglichkeit, insbesondere eine hohe Verträglichkeit gegenüber Kohlenwasserstoffen, auf und lassen sich zu Produkten mit einem hohen Treibgasgehalt von z. B. mindestens 40 Gew.-%, bevorzugt mindestens 50 Gew.-%, bezogen auf das Gesamtgewicht des Mittels, formulieren. Im Allgemeinen ist es aber auch möglich, den Treibmittelgehalt gering zu halten, um Produkte mit einem niedrigen VOC-Gehalt zu formulieren. In solchen Produkten beträgt der Treibgasgehalt dann im Allgemeinen nicht mehr als 55 Gew.-%, bezogen auf das Gesamtgewicht des Mittels. Die erfindungsgemäßen Haarfestigungsmittel eignen sich auch für Pumpsprayzubereitungen ohne den Zusatz von Treibmitteln.

Die zuvor beschriebenen Polymere können auch in Kombination mit anderen Haarpolymeren in den Mitteln zur Anwendung kommen. Solche Polymere sind insbesondere:
- nichtionische, wasserlösliche bzw. wasserdispergierbare Polymere oder Oligomere, wie Polyvinylcaprolactam, z. B. Luviskol Plus (BASF), oder Polyvinylpyrrolidon und deren Copolymere, insbesondere mit Vinylestern, wie Vinylacetat, z. B. Luviskol VA 37 (BASF); Polyamide, z. B. auf Basis von Itaconsäure und aliphatischen Diaminen;
- amphotere oder zwitterionische Polymere, wie die unter den Bezeichnungen Amphomer® (Delft National) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind und Copolymere aus Hydroxyethylmethacrylat, Methylmethacrylat, N,N-Dimethylaminoethylmethacrylat und Acrylsäure (Jordapon®);
- anionische Polymere, wie Vinylacetat/Crotonsäure-Copolymere, wie sie beispielsweise unter den Bezeichnungen Resyn® (NATIONAL STARCH), Luviset® (BASF) und Gafset® (GAF) im Handel sind, Vinylpyrrolidon/Vinylacrylat-Copolymere, erhältlich beispielsweise unter dem Warenzeichen Luviflex® (BASF). Ein bevorzugtes Polymer ist das unter der Bezeichnung Luviflex® VBM-35 (BASF) erhältliche Vinylpyrrolidon/Acrylat-Terpolymer, Acrylsäure/Ethylacrylat/N-tert.Butylacrylamid-Terpolymere, die beispielsweise unter der Bezeichnung Ultrahold® strong (BASF) vertrieben werden, sowie Luvimer® (BASF, Terpolymer aus t-Butylacrylat, Ethylacrylat und Methacrylsäure), oder
- kationische (quaternisierte) Polymere, z. B. kationische Polyacrylatcopolymere auf Basis von N-Vinyllactamen und deren Derivaten (N-Vinylpyrrolidon, N-Vinylcaprolactam etc.) sowie übliche kationische Haarconditionerpolymere, z. B. Luviquat® (Copolymer aus Vinylpyrrolidon und Vinylimidazoliummethochlorid), Luviquat® Hold (Copolymerisat aus quaternisiertem N-Vinylimidazol, N-Vinylpyrrolidon und N-Vinylcaprolactam), Merquat® (Polymer auf Basis von Dimethyldiallylammoniumchlorid), Gafquat® (quaternäre Polymere, die durch Reaktion von Polyvinylpyrrolidon mit quaternären Ammoniumverbindungen entstehen), Polymer JR (Hydroxyethylcellulose mit kationischen Gruppen), Polyquaternium-Typen (CTFA-Bezeichnungen) etc.;
- nichtionische, siloxanhaltige, wasserlösliche oder -dispergierbare Polymere, z. B. Polyethersiloxane, wie Tegopren® (Fa. Goldschmidt) oder Belsil® (Fa. Wacker).

Die erfindungsgemäßen Polymere können mit unvernetzten und vernetzten siloxangruppenhaltigen Polyurethanen und/oder mit wenigstens einem anderen siloxanfreien amidgruppenhaltigen Haarpolymer eingesetzt werden. Dazu zählen z. B. die in der DE-A-42 25 045 beschriebenen Polyurethane, die zuvor beschriebenen Vinylpyrrolidon/Acrylat-Terpolymere und Acrylsäure/Ethylacrylat/N-tert.-Butylacrylamid-Terpolymere (z. B. Ultrahold®strong der BASF AG), die in der DE-A-42 41 118 beschriebenen kationischen Polyurethane, die zuvor beschriebenen amidgruppenhaltigen amphoteren Polymere (z. B. Amphomer®) und insbesondere Copolymerisate, die einen Anteil an amidgruppenhaltigen Monomeren, wie N-Vinyllactamen, von mindestens 30 Gew.-% aufweisen (z. B. Luviskol®plus und Luviskol®VA37 der BASF AG).

Die anderen Haarpolymere sind vorzugsweise in Mengen bis zu 10 Gew.-%, bezogen auf das Gesamtgewicht des Mittels enthalten.

Ein bevorzugtes Haarbehandlungsmittel enthält:
A) 0,2 bis 20 Gew.-% mindestens eines in Wasser löslichen oder dispergierbaren Polymers, wie in einem der Ansprüche 1 bis 5 definiert,
B) 30 bis 99,5 Gew.-%, bevorzugt 40 bis 99 Gew.-%, wenigstens eines Lösungsmittels, ausgewählt unter Wasser, wassermischbaren Lösungsmitteln und Mischungen davon,
C) 0 bis 70 Gew.-% eines Treibmittels,
D) 0 bis 10 Gew.-% mindestens eines von A) verschiedenen, in Wasser löslichen oder dispergierbaren Haarpolymers,
E) 0 bis 0,3 Gew.-% mindestens eines wasserunlöslichen Silicons,
F) 0 bis 0,5 Gew.-% mindestens eines Wachses, insbesondere eines Fettsäureamids,
sowie übliche Zusatzstoffe.

Das erfindungsgemäße Mittel kann als Komponente D) mindestens ein anderes, in Wasser lösliches oder dispergierbares Haarpolymer enthalten. Der Anteil dieser Komponente beträgt dann im Allgemeinen etwa 0,1 bis 10 Gew.-%, bezogen auf das Gesamtgewicht des Mittels. Bevorzugt können dabei wasserlösliche oder wasserdispergierbare Polyurethane eingesetzt werden, die gewünschtenfalls zusätzlich Siloxangruppen einpolymerisiert enthalten.

Das erfindungsgemäße Mittel kann als Komponente E) mindestens ein wasserunlösliches Silicon, insbesondere ein Polydimethylsiloxan, z. B. die Abil® -Typen der Fa. Goldschmidt, enthalten. Der Anteil dieser Komponente beträgt dann im Allgemeinen etwa 0,0001 bis 0,2 Gew.-%, bevorzugt 0,001 bis 0,1 Gew.-%, bezogen auf das Gesamtgewicht des Mittels.

Bevorzugt wird als Komponente F) wenigstens ein Fettsäureamid, wie z. B. Erucasäureamid, eingesetzt.

Das erfindungsgemäße Mittel kann zusätzlich gegebenenfalls einen Entschäumer, z. B. auf Silicon-Basis, enthalten. Die Menge des Entschäumers beträgt im Allgemeinen bis zu etwa 0,001 Gew.-%, bezogen auf die Gesamtmenge des Mittels.

Die erfindungsgemäßen Mittel besitzen den Vorteil, dass sie einerseits den Haaren die gewünschte Festigkeit verleihen und andererseits die Polymere leicht auswaschbar (redispergierbar) sind. Dem Haar wird im Allgemeinen ein natürliches Aussehen und Glanz verliehen, auch wenn es sich um von Natur aus besonders kräftiges und/oder dunkles Haar handelt.

Insbesondere lassen sich die erfindungsgemäßen Mittel zu Haarbehandlungsmitteln, insbesondere Haarsprays, mit einem hohen Treibstoffgehalt formulieren. Vorteilhafterweise weisen die erfindungsgemäßen Haarbehandlungsmitteln im Wesentlichen keinen "Flaking"-Effekt auf.

Die Erfindung wird anhand der folgenden, nicht einschränkenden Beispiele näher erläutert.

### Beispiele

### Vergleichsbeispiele A-C, Beispiele 1-25:

### Lösungspolymerisation (Beispiel 13)

- Zulauf 1:: 640 g Monomerengemisch aus 590 g Vinylpyrrolidon und 50 g N-[3-(dimethylamino)propyl]acrylamid (DMAPMA)
- Zulauf 2:: 350 g Monomerengemisch aus 250 g tert.-Butylacrylat, 50 g N,N-Dimethylaminopropylacrylamid und 50 g Stearylmethacrylat
- Zulauf 3:: 2,5 g 2,2'-Azobis(2-methylbutyronitril) in 300 g Ethanol
- Zulauf 4:: 7,5 g 2,5-Bis(tert.-butylperoxy-2,5-dimethylhexan) (Trigonox® 101 der Fa. Akzo Nobel) in 500 g Ethanol
- Zulauf 5:: 104 g Phosphorsäure (50 %ig in Ethanol)

In einer Rührapparatur mit Rückflusskühler und drei separaten Zulaufvorrichtungen wurden 128 g von Zulauf 1, 35 g von Zulauf 2, 20 g von Zulauf 3 und 10 g ethoxiliertes Polysiloxan (Belsil® DMC 6031 der Fa. Wacker) und 200 g Ethanol vorgelegt und die Mischung unter Rühren auf ca. 75 °C aufgeheizt. Nach dem Anpolymerisieren, erkennbar an einer beginnenden Viskositätserhöhung, wurde der Rest von Zulauf 1 innerhalb von einer Stunde, der Rest von Zulauf 2 innerhalb von vier Stunden und der Rest von Zulauf 3 innerhalb von fünf Stunden zugegeben, wobei die Innentemperatur auf ca. 80 °C erhöht wurde. Nach dem Ende der Zugabe wurde noch ca. zwei Stunden bei dieser Temperatur nachpolymerisiert.

Zur Verringerung des Restmonomerengehaltes wird anschließend der Zulauf 4 innerhalb von zwei Stunden zugegeben und die Reaktionsmischung 10 Stunden bei 130 °C unter Eigendruck nachpolymerisiert. (Alternativ kann man den Restmonomergehalt auch durch Zugabe von Wasser und Phosphorsäure und anschließendes Erwärmen auf Temperaturen bis 100 °C erreichen, wobei man die Phosphorsäure in einer Menge zugibt, bis ein pH-Wert im Bereich von 4,5 bis 5,5 erreicht ist (entsprechend einer Neutralisation des eingesetzten DMAPMA von 80 bis 120 Mol-%). Vorzugsweise führt man dann eine Wasserdampfdestillation durch, bis eine Innentemperatur von etwa 100 °C erreicht ist.)

Nach dem Abkühlen neutralisiert man den Ansatz durch Zugabe von Zulauf 5 innerhalb von 30 Minuten unter Rühren.

Die anwendungstechnischen Eigenschaften der so erhaltenen Polymere sind in Tabelle 3 wiedergegeben.

Die ethanolischen Produktlösungen können gewünschtenfalls nach der Neutralisation mit Wasser versetzt und der Alkohol kann durch Abdestillieren entfernt werden. Dabei werden wässrige (Mikro)dispersionen erhalten. Pulverförmige Produkte können durch Sprühtrocknen oder Gefriertrocknen erhalten werden.

Analog dieser Herstellungsvorschrift wurden auch die Polymere A-C, 1-12 und 14-33 hergestellt.

Alle Polymere der folgende Tabelle 1 weisen K-Werte (gemessen in 1 %iger ethanolischer Lösung) im Bereich von 37 bis 45 auf.

**Tabelle 1:**

| Bsp Nr. | TBA ¹⁾ | nBA ²⁾ | SMA ³⁾ | LA ⁴⁾ | MAS ⁵⁾ | VP ⁶⁾ | VFA ⁷⁾ | DMA PMA ⁸⁾ | Siloxan ⁹⁾ | AA ¹⁰⁾ | VI ¹¹⁾ |
|---|---|---|---|---|---|---|---|---|---|---|---|
| A | -- | 25 | -- | -- | -- | 65 | -- | 10*⁾ | -- | -- | -- |
| B | -- | -- | 10 | -- | -- | 80 | -- | 10**⁾ | -- | -- | -- |
| C | -- | -- | 30 | -- | -- | 60 | -- | 10**⁾ | -- | -- | -- |
| 1 | 10 | -- | -- | -- | -- | 80 | -- | 10**⁾ | -- | -- | -- |
| 2 | 25 | -- | -- | -- | -- | 65 | -- | 10*⁾ | -- | -- | -- |
| 3 | 30 | -- | -- | -- | -- | 60 | -- | 10**⁾ | -- | -- | -- |
| 4 | 15 | -- | 5 | -- | -- | 65 | -- | 15*⁾ | -- | -- | -- |
| 5 | 15 | -- | -- | 5 | -- | 64 | -- | 15*⁾ | 1 | -- | -- |
| 6 | 25 | -- | -- | -- | -- | 65 | -- | 10*⁾ | -- | -- | -- |
| 7 | 25 | -- | -- | -- | -- | 55 | -- | 20*⁾ | -- | -- | -- |
| 8 | 25 | -- | -- | -- | -- | 55 | -- | 17*⁾ | 3 | -- | -- |
| 9 | 25 | 10 | -- | -- | -- | 50 | -- | 15*⁾ | -- | -- | -- |
| 10 | 30 | -- | -- | -- | -- | 60 | -- | 10*⁾ | -- | -- | -- |
| 11 | 30 | -- | -- | -- | -- | 50 | -- | 20*⁾ | -- | -- | -- |
| 12 | 30 | -- | -- | -- | -- | 59 | -- | 10*⁾ | 1 | -- | -- |
| 13 | 25 | -- | -- | 5 | -- | 59 | -- | 10*⁾ | 1 | -- | -- |
| 14 | 40 | -- | -- | -- | 6 | 40 | -- | 12*⁾ | 2 | -- | -- |
| 15 | 40 | -- | -- | -- | -- | 50 | -- | 10*⁾ | -- | -- | -- |
| 16 | 40 | -- | -- | -- | -- | 50 | -- | 10**⁾ | -- | -- | -- |
| 17 | 40 | -- | -- | -- | -- | 49,5 | -- | 10**⁾ | 0,5 | -- | -- |
| 18 | 49 | -- | -- | -- | -- | 35 | -- | 15*⁾ | 1 | -- | -- |
| 19 | 49 | -- | -- | -- | 5 | 30 | -- | 15*⁾ | 1 | -- | -- |
| 20 | 30 | 19 | -- | -- | 5 | 27 | -- | 17*⁾ | 2 | -- | -- |
| 21 | 20 | -- | -- | -- | -- | 70 | -- | 10*⁾ | -- | -- | -- |
| 22 | 20 | -- | -- | -- | 4 | 65 | -- | 8*⁾ | 3 | -- | -- |
| 23 | 20 | -- | -- | -- | 3,5 | -- | 70 | 6,5*⁾ | -- | -- | -- |
| 24 | 30 | -- | -- | -- | 7 | -- | 50 | 13*⁾ | -- | -- | -- |
| 25 | 32 | -- | -- | -- | 8 | -- | 40 | 16*⁾ | 4 | -- | -- |
| 26 | 40 | -- | -- | -- | 5 | 38 | -- | 10 | 7 | -- | -- |
| 27 | 40 | -- | -- | -- | 4/3 ***⁾ | 38 | -- | 8 | 7 | -- | -- |
| 28 | 32 | -- | 8 | -- | 3/9 ***⁾ | 35 | -- | 6 | 7 | -- | -- |
| 29 | 30 | -- | -- | -- | 5 | 20 | -- | 10 | 5 | -- | -- |
| 30 | 40 | -- | -- | -- | 4/3 ***⁾ | 20 | -- | 8 | 7 | 18 | -- |
| 31 | 20 | -- | -- | -- | 2/10 ***⁾ | 20 | -- | 4 | 4 | 40 | -- |
| 32 | 20 | -- | -- | -- | 5 | 20 | -- | 10 | 4,5 | -- | -- |
| 33 | 10 | -- | -- | -- | 9 | 60 | -- | -- | -- | 70,7 | 10 |

| | | | | | | | | | | | |
|---|---|---|---|---|---|---|---|---|---|---|---|
| *⁾ neutralisiert mit H₃PO₄, Neutralisationsgrad ca. 90 % | | | | | | | | | | | |
| **⁾ anstelle der Zugabe von Phosphorsäure wurde das einpolymerisierte DMAPMA durch Zugabe von 0,9 bis 1 Äquivalenten Diethylsulfat, bezogen auf DMAPMA, bei 40 °C (ca. 1 h) quaternisiert. | | | | | | | | | | | |
| ***⁾ nicht neutralisiert/neutralisiert mit KOH | | | | | | | | | | | |
| 1) TBA = tert.-Butylacrylat | | | | | | | | | | | |
| 2) nBA = n-Butylacrylat | | | | | | | | | | | |
| 3) SMA = Stearylmethacrylat | | | | | | | | | | | |
| 4) LA = Laurylacrylat | | | | | | | | | | | |
| 5) MAS = Methacrylsäure | | | | | | | | | | | |
| 6) VP = N-Vinylpyrrolidon | | | | | | | | | | | |
| 7) VFA = N-Vinylformamid | | | | | | | | | | | |
| 8) DMAPMA = N-[3-(dimethylamino)propyl]acrylamid | | | | | | | | | | | |
| 9) Siloxan = ethoxiliertes Polysiloxan (Belsil@ DMC 6031 der Fa. Wacker) | | | | | | | | | | | |
| 10) AA = Acrylamid | | | | | | | | | | | |
| 11) VI = Vinylimidazol | | | | | | | | | | | |

### Anwendungstechnische Eigenschaften

An den Polymeren aus den Vergleichsbeispielen A-C sowie den erfindungsgemäßen Beispielen 1-25 wurde die n-Heptan-Verträglichkeit als Maß für ihre Treibmittelverträglichkeit bestimmt. Dazu wurden je 1,5 g neutralisiertes Polymer und 23,5 g Ethanol zu 6 gew.-%igen Lösungen formuliert und bei Raumtemperatur (22 °C) mit n-Heptan titriert, bis eine Trübung auftritt. Die prozentuale n-Heptan-Verträglichkeit wurde in eine Notenskala gemäß Tabelle 2 umgesetzt.

Die Polymere aus den Vergleichsbeispielen A-C sowie aus den erfindungsgemäßen Beispielen 1-25 wurden zu 5 gew.-%igen ethanolischen Lösungen formuliert. Sie wurden auf eine Glasplatte aufgetragen und die resultierenden Filme wurden im Hinblick auf drei Kriterien (Auswaschbarkeit, Klebrigkeit und Griff), die in Tabelle 2 angegeben sind, von fünf Fachleuten geprüft und mit Noten von 1 bis 4 bewertet. Die Bewertungen der Filme sind in Tabelle 3 wiedergegeben. Ebenso wurde die Stärke der Festigung mit Hilfe eines Modellkopfes bewertet.

**Tabelle 2:**

| Note | Heptan-Verträglichkeit | Festigung | Auswaschbarkeit | Klebrigkeit | Griff |
|---|---|---|---|---|---|
| 1 | > 65 % | sehr gut | sehr gut | nicht klebrig | sehr geschmeidig |
| 2 | 50-65 % | gut | gut | leicht klebrig | gut, geschmeidig |
| 3 | 35-50 % | mäßig | mäßig | klebrig | rauh, stumpf |
| 4 | < 35 % | schlecht | schlecht löslich | sehr klebrig | rauh, bremsend, unnatürlich |

**Tabelle 3:**

| Bsp. Nr. | Note | | | | |
|---|---|---|---|---|---|
| | Heptan-Verträglichkeit | Festigung | Auswaschbarkeit | Klebrigkeit | Griff |
| A | 2 | 2 | 1-2 | 2 | 2-3 |
| B | 1 | 2 | 1-2 | 2 | 3 |
| C | 1 | 2-3 | 3 | 2-3 | 3 |
| 1 | 1 | 1-2 | 1 | 2 | 1-2 |
| 2 | 1 | 1-2 | 1-2 | 1-2 | 2 |
| 3 | 1 | 1 | 1 | 1-2 | 2 |
| 4 | 1 | 1 | 1-2 | 1-2 | 2 |
| 5 | 1 | 1-2 | 1-2 | 1-2 | 1-2 |
| 6 | 1 | 1 | 1 | 1-2 | 2 |
| 7 | 2 | 1 | 1 | 1-2 | 2 |
| 8 | 1-2 | 1-2 | 1 | 1-2 | 1-2 |
| 9 | 1 | 1-2 | 1 | 1-2 | 2 |
| 10 | 1 | 1 | 1 | 1-2 | 2 |
| 11 | 1-2 | 1 | 1 | 1 | 2 |
| 12 | 1 | 1 | 1 | 1-2 | 1-2 |
| 13 | 1 | 1 | 1 | 1-2 | 1-2 |
| 14 | 1 | 1 | 1 | 1 | 1-2 |
| 15 | 1 | 1 | 1-2 | 1 | 2 |
| 16 | 1 | 1 | 1 | 1-2 | 1-2 |
| 17 | 1 | 1 | 1 | 1-2 | 1-2 |
| 18 | 1 | 1 | 1 | 1 | 1-2 |
| 19 | 1 | 1 | 1 | 1 | 1-2 |
| 20 | 1-2 | 1-2 | 1 | 1-2 | 1 |
| 21 | 1-2 | 2 | 1 | 2 | 2 |
| 22 | 1-2 | 2 | 1 | 1-2 | 1-2 |
| 23 | 2 | 2*⁾ | 1 | 2 | 1-2 |
| 24 | 1-2 | 1-2*⁾ | 1 | 1-2 | 1-2 |
| 25 | 1-2 | 1-2*⁾ | 1 | 1-2 | 1 |

| | | | | | |
|---|---|---|---|---|---|
| *⁾ gemessen an einem Film einer Haarspray-Formulierung mit einem VOC-Gehalt von 80 Gew.-% (Treibgas: Dimethylether) | | | | | |

### Beispiele 26 bis 50

Aerosol-Haarspray-Formulierungen mit einem VOC-Gehalt von 95 Gew.-%:

| | |
|---|---|
| Polymer gemäß Beisp. 1-25 | 5,00 Gew.-% |
| Ethanol | 55,00 Gew.-% |
| Propan/Butan | 39,96 Gew.-% |
| Parfüm, Zusatzstoffe | q.s. |

### Beispiele 51 bis 75

Aerosol-Haarspray-Formulierungen mit einem VOC-Gehalt von 95 Gew.-%:

| | |
|---|---|
| Polymer gemäß Beisp. 1-25 | 5,00 Gew.-% |
| Ethanol | 40,00 Gew.-% |
| Propan/Butan | 54,96 Gew.-% |
| Parfüm, Zusatzstoffe | q.s. |

### Beispiele 76 bis 100

Kompakte Aerosol-Haarspray-Formulierungen mit einem VOC-Gehalt von 80 Gew.-%:

| | |
|---|---|
| Polymer gemäß Beisp. 1-25 | 5,00 Gew.-% |
| Ethanol | 40,00 Gew.-% |
| Dimethylether | 39,96 Gew.-% |
| Wasser | 15,00 Gew.-% |
| Parfüm, Zusatzstoffe | q.s. |

### Beispiele 101 bis 117

Haarspray-Formulierungen mit einem VOC-Gehalt von 55 Gew.-%:
Polymer gemäß Beisp.

| | |
|---|---|
| 1-3,7-14,16-21 | 3,00 Gew.-% |
| Ethanol | 15,00 Gew.-% |
| Wasser | 42,00 Gew.-% |
| Dimethylether | 39,96 Gew.-% |
| Parfüm, Zusatzstoffe | q.s. |

### Beispiele 118 bis 142

Pump-Haarspray:

| | |
|---|---|
| Polymer gemäß Beisp. 1-25 | 5,00 Gew.-% |
| Ethanol | 94,96 Gew.-% |
| Parfüm, Zusatzstoffe | q.s. |

### Beispiele 143 bis 167

Pump-Haarspray:

| | |
|---|---|
| Polymer gemäß Beisp. 1-25 | 5,00 Gew.-% |
| Ethanol | 79,96 Gew.-% |
| Wasser | 15,00 Gew.-% |
| Parfüm, Zusatzstoffe | q.s. |

### Beispiele 168 bis 192

| Schaum-Conditioner : | [Gew.-%] |
|---|---|
| Polymer 1-25 (20 %ige wässrige | |
| Lösung) | 30,00 |
| Cremophor® A25⁸⁾ | 0,20 |
| Comperlan® KD⁹⁾ | 0,10 |
| Wasser | 59,70 |
| Propan/Butan | 9,96 |
| Parfüm, Konservierungsmittel | q.s. |

| | |
|---|---|
| ⁸⁾ CTFA-Name: Ceteareth 25, Fa. BASF AG, Umsetzungsprodukt aus Fettalkohol und Ethylenoxid | |
| ⁹⁾ CTFA-Name: Cocamide DEA, Fa. Henkel, Kokosfettsäureamid | |

Zur Herstellung der Schaum-Conditioner werden die Komponenten eingewogen und unter Rühren gelöst. Anschließend werden sie in einen Spender abgefüllt und das Treibgas zugesetzt.

### Beispiele 193 bis 217

### Haarfestiger-Gel

### Phase I:

| | |
|---|---|
| Polymer 1-25 (20 %ige wässrige | |
| Lösung) | 25,00 Gew.-% |
| Polysorbat 20 | 1,00 Gew.-% |
| Imidazolidinylharnstoff | 0,10 Gew.-% |
| Parfüm, Zusatzstoffe | q.s. |

### Phase II:

| | |
|---|---|
| Hydroxyethylcellulose | 1,40 Gew.-% |
| Ethoxilierter Oleylalkohol (20 EO) | 0,10 Gew.-% |
| Wasser | 72,36 Gew.-% |

### Beispiele für Anwendungen in der Hautkosmetik

### Beispiele 218 bis 242

### O/W-Cremes

| Ölphase: | Gew.-% | CTFA-Name: |
|---|---|---|
| Cremophor® A6 (BASF AG) | 3,5 | Ceteareth-6 (Stearylalkohol-Ethoxylat) |
| Cremophor® A25 (BASF AG) | 2,5 | Ceteareth-25 (Fettalkohol-Ethoxylat) |
| Glycerinmonostearat s.e. | 2,5 | Glycerylstearate |
| Paraffinöl | 7,5 | |
| Cetylalkohol | 2,5 | |
| Luvitol® EHO (BASF AG) | 3,2 | Cetearyloctanoat |
| Vitamin E-Acetat | 1,0 | |
| Nip-Nip®, Nipa Laboratories Ltd., USA | 0,1 | Methyl- und Propyl-4-hydroxybenzoate (7:3) |

| Wasserphase: | Gew.-% | |
|---|---|---|
| Polymer 1-25 | 1,5 | |
| Wasser | 73,6 | |
| Germall II, Sutton Laboratories Inc., USA | 0,1 | Imidazolidinylharnstoff |
| 1,2-Propylenglykol | 1,0 | |

Zur Herstellung der Cremes werden die Komponenten für Öl- und Wasserphase getrennt eingewogen und bei 80 °C homogenisiert. Dann gibt man die wasserphase langsam unter Rühren zu der Ölphase. Anschließend lässt man unter Rühren auf Raumtemperatur abkühlen.

### Beispiele 243 bis 267

### O/W-Cremes

| Ölphase: | Gew.-% | CTFA-Name: |
|---|---|---|
| Cremophor® A6 (BASF AG) | 2,0 | Ceteareth-6 (Stearylalkohol-Ethoxylat) |
| Cremophor® A25 (BASF AG) | 2,0 | Ceteareth-25 (Fettalkohol-Ethoxylat) |
| Glycerinmonostearat s.e. | 6,0 | Glycerylstearate |
| Paraffinöl | 0,9 | |
| Tegiloxan 100 | 0,1 | Dimethicone |
| Cetylalkohol | 1,5 | |
| Luvitol® EHO (BASF AG) | 12,0 | Cetearyloctanoat |
| Vitamin E-Acetat | 0,4 | |

| Wasserphase: | Gew.-% | |
|---|---|---|
| Polymer 1-25 | 1,0 | |
| Wasser | 74,6 | |
| 1,2-Propylenglykol | 1,0 | |
| Germall II, Sutton Laboratories Inc., USA | 0,1 | Imidazolidinylharnstoff |

Zur Herstellung der Cremes werden die Komponenten für Öl- und Wasserphase getrennt eingewogen und bei 80 °C homogenisiert. Dann gibt man die Wasserphase langsam unter Rühren zu der Ölphase. Anschließend lässt man unter Rühren auf Raumtemperatur abkühlen.

Beispiele für Verwendung als Haarfestigerpolymer in der Haarkosmetik

### Beispiele 268-277

| VOC 80 Aerosol-Haarspray | [Gew.-%] |
|---|---|
| Polymer 14/19/20/22/25/26-30 | 4,00 |
| Luviset® PUR (Polyurethan- | |
| Haarpolymer der Fa. BASF) | 1,00 |
| Wasser | 15,00 |
| Dimethylether | 40,00 |
| Ethanol | 40,00 |
| Silikon, Parfüm, Entschäumer | q.s. |

### Beispiele 278-287

| VOC 80 Aerosol-Haarspray | [Gew.-%] |
|---|---|
| Polymer 14/19/20/22/25/26-30 | 1,50 |
| Luviset PUR | 3,50 |
| Wasser | 15,00 |
| Dimethylether | 40,00 |
| Ethanol | 40,00 |
| Silikon, Parfüm, Entschäumer | q.s. |

### Beispiele 288-297

| VOC 55 Aerosol-Haarspray | [Gew.-%] |
|---|---|
| Polymer 14/19/20/22/25/26-30 | 1,00 |
| Luviset PUR | 2,00 |
| Wasser | 42,00 |
| Dimethylether | 35,00 |
| Ethanol | 20,00 |
| Silikon, Parfüm, Entschäumer | q.s. |

### Beispiele 298-307

| VOC 55 Handpumpen-Spray | [Gew.-%] |
|---|---|
| Polymer 14/19/20/22/25/26-30 | 1,50 |
| Luviset PUR | 3,50 |
| Wasser | 40,00 |
| Ethanol | 55,00 |
| Silikon, Parfüm, Entschäumer | q.s. |

### Beispiele 308-312

| Schaum-Conditioner | [Gew.-%] |
|---|---|
| Polymer 26-30 (25%ige wässrige | |
| Lösung) | 20,00 |
| Cremophor® A25 (Ceteareth-25/BASF) | 0,20 |
| Comperlan® KD (Coamide DEA/Henkel) | 0,10 |
| Wasser | 69,70 |
| Propan/Butan | 10,00 |
| Silikon, Parfüm, Konservierungsmittel | q.s. |

Herstellung: Die Komponenten werden eingewogen, unter Rühren gelöst, abgefüllt und anschließend das Treibgas zugesetzt.

### Beispiele 313-315

| Conditioner-Shampoo | | [Gew.-%] |
|---|---|---|
| Polymer 26-30 | | |
| (25 | % wässrige Lösung) | 20,00 |
| A) | Texapon® NSO 28%ig | |
| | (Natriumlaurylsulfat/Henkel) | 50,00 |
| | Comperlan® KD | |
| | (Coamide DEA/Henkel) | 1,00 |
| | Polymer 31-33 | |
| | (25%ige wässrige Lösung) | 20,00 |
| | Parfümöl | q.s. |
| B) | Wasser | 27,50 |
| | Natriumchlorid | 1,50 |
| | Konservierungsmittel etc. | q.s. |

Herstellung: Die Komponenten werden eingewogen, die Phasen A und B unter Rühren getrennt gelöst und gemischt. Dann rührt man Phase B langsam in Phase A ein.

### Verwendung in der Hautkosmetik

### Beispiele 316-328

### Standard O/W-Cremes

| Ölphase: | Gew.-% | CTFA-Name: |
|---|---|---|
| Cremophor® A6 (BASF AG) | 3,5 | Ceteareth-6 (und) Stearylalkohol |
| Cremophor® A25 (BASF AG) | 3,5 | Ceteareth-25 |
| Glycerinmonostearat s.e. | 2,5 | Glycerylstearat |
| Paraffinöl | 7,5 | |
| Cetylalkohol | 2,5 | |
| Luvitol® EHO (BASF AG) | 3,2 | Cetearyloctanoat |
| Vitamin E-Acetat | 1,0 | Tocopherylacetat |
| NiP-Nip®, Nipa Lab. Ltd., USA | 0,1 | Methyl- und Propyl-4-hydroxybenzoat (7:3) |

| Wasserphase: | Gew.-% | |
|---|---|---|
| Polymer 14/19/20/22/25/26-33 | 1,5 | |
| Wasser | 73,6 | |
| 1,2-Propylenglykol | 1,0 | |
| Germall II, Sutton Lab.Inc., USA | 0,1 | Imidazolidinylharnstoff |

Zur Herstellung der Cremes werden die Komponenten für Öl- und Wasserphase getrennt eingewogen und bei 80 °C homogenisiert. Dann gibt man die Wasserphase langsam unter Rühren zu der Ölphase. Anschließend lässt man unter Rühren auf Raumtemperatur abkühlen.

## Patentansprüche

1. Kosmetisches Mittel, enthaltend wenigstens ein wasserlösliches oder wasserdispergierbares Polymer, das
a) 5 bis weniger als 50 Gew.-% wenigstens eines α,β-ethylenisch ungesättigten Monomers der allgemeinen Formel I 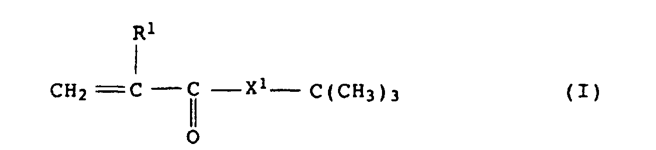 worin
R¹ für Wasserstoff oder C₁- bis C₈-Alkyl steht, und
X¹ für O oder NR² steht, wobei R² für Wasserstoff, C₁- bis C₈-Alkyl oder C₅- bis C₈-Cycloalkyl steht,
b) 25 bis 90 Gew.-% wenigstens eines N-Vinylamids und/oder N-Vinyllactams,
c) 0,5 bis 30 Gew.-% wenigstens einer Verbindung mit einer radikalisch polymerisierbaren, α,β-ethylenisch ungesättigten Doppelbindung und mindestens einer kationogenen und/oder kationischen Gruppe pro Molekül,
d) 0 bis 30 Gew.-% wenigstens eines α,β-ethylenisch ungesättigten Monomers der allgemeinen Formel II 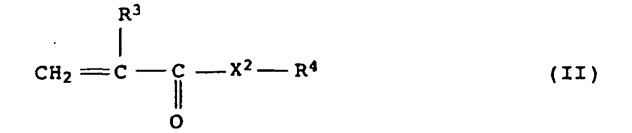 worin
R³ für Wasserstoff oder C₁- bis C₈-Alkyl steht,
X² für O oder NR⁵ steht, wobei R⁵ für Wasserstoff, C₁ bis C₈-Alkyl oder C₅- bis C₈-Cycloalkyl steht, und
R⁴ für Wasserstoff oder einen linearen C₁- bis C₂₂-Alkylrest steht,
eingebaut enthält, und die Salze davon.

2. Mittel nach Anspruch 1, wobei das wasserlösliche oder wasserdispergierbare Polymer wenigstens ein Monomer d) der Formel II mit X² = O und R⁴ = H in einer Menge von 0,1 bis 20 Gew.-% bezogen auf die Gesamtmenge der Monomere a), b), c) und d) einpolymerisiert enthält.

3. Mittel nach einem der vorhergehenden Ansprüche, wobei das Polymer wenigstens eine weitere Komponente, die ausgewählt ist unter
e) von d) verschiedenen Verbindungen mit einer radikalisch polymerisierbaren α,β-ethylenisch ungesättigten Doppelbindung und mindestens einer anionogenen und/oder anionischen Gruppe pro Molekül,
f) vernetzend wirkenden Monomeren mit mindestens zwei ethylenisch ungesättigten, nicht konjugierten Doppelbindungen,
g) von a) bis f) verschiedenen radikalisch polymerisierbaren Monomeren,
und Mischungen davon, einpolymerisiert enthält.

4. Mittel nach einem der vorhergehenden Ansprüche, das zusätzlich wenigstens ein Polyalkylenoxidgruppen-haltiges Polysiloxan enthält.

5. Mittel nach Anspruch 4, wobei das Polysiloxan ausgewählt ist unter Verbindungen der allgemeinen Formel III 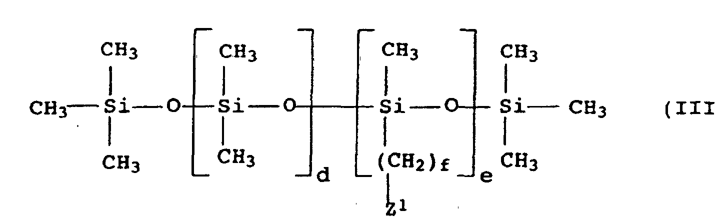 worin
die Reihenfolge der Siloxaneinheiten beliebig ist,
d und e unabhängig voneinander für 0 bis 100 stehen, wobei die Summe aus d und e mindestens 2 ist,
f für eine ganze Zahl von 2 bis 8 steht,
Z¹ für einen Rest der Formel IV
-(CH₂)ᵤ-O-(CH₂CH₂O)ᵥ(CH₂CH(CH₃)O)_{w}-R^{A} (IV)
steht, wobei
in der Formel IV die Reihenfolge der Alkylenoxideinheiten beliebig ist,
u für eine ganze Zahl von 1 bis 8 steht,
v und w unabhängig voneinander für eine ganze Zahl von 0 bis 200 stehen, wobei die Summe aus v und w > 0 ist, und
R^{A} für Wasserstoff oder C₁-C₆-Alkyl steht.

6. Mittel nach einem der Ansprüche 4 oder 5, wobei das Gewichtsmengenverhältnis von wasserlöslichem oder wasserdispergierbarem Polymer zu Polysiloxan in einem Bereich von 70:30 bis 99,9:0,1, bevorzugt 85:15 bis 99:1, liegt.

7. Mittel nach einem der Ansprüche 4 bis 6, wobei als wasserlösliches oder wasserdispergierbares Polymer ein Polymer eingesetzt wird, das durch radikalische Polymerisation der das Polymer bildenden Monomere in Gegenwart von wenigstens einem der Polyalkylenoxidgruppen-haltigen Polysiloxane erhalten wird.

8. Mittel nach einem der vorhergehenden Ansprüche in Form eines Haarbehandlungsmittels, insbesondere in Form eines Haarsprays.

9. Mittel nach Anspruch 8, enthaltend
A) 0,2 bis 20 Gew.-% mindestens eines in Wasser löslichen oder dispergierbaren Polymers, wie in einem der Ansprüche 1 bis 6 definiert,
B) 30 bis 99,5 Gew.-%, bevorzugt 40 bis 99 Gew.-%, wenigstens eines Lösungsmittels, ausgewählt unter Wasser, wassermischbaren Lösungsmitteln und Mischungen davon,
C) 0 bis 70 Gew.-% eines Treibmittels,
D) 0 bis 10 Gew.-% mindestens eines von A) verschiedenen, in Wasser löslichen oder dispergierbaren Haarpolymers,
E) 0 bis 0,3 Gew.-% mindestens eines wasserunlöslichen Silicons,
F) 0 bis 0,5 Gew.-% mindestens eines Wachses, bevorzugt mindestens eines Fettsäureamids.

10. Wasserlösliches oder wasserdispergierbares Polymer, das
a) 5 bis weniger als 50 Gew.-% wenigstens eines α,β-ethylenisch ungesättigten Monomers der allgemeinen Formel I 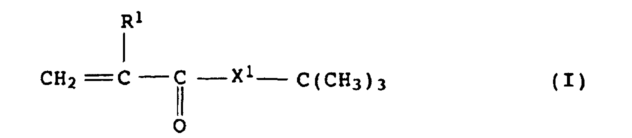 worin
R¹ für Wasserstoff oder C₁- bis C₈-Alkyl steht, und
X¹ für O oder NR² steht, wobei R² für Wasserstoff, C₁- bis C₈-Alkyl oder C₅- bis C₈-Cycloalkyl steht,
b) 25 bis 90 Gew.-% wenigstens eines N-Vinylamids und/oder N-Vinyllactams,
c) 0,5 bis 30 Gew.-% wenigstens einer Verbindung mit einer radikalisch polymerisierbaren, α,β-ethylenisch ungesättigten Doppelbindung und mindestens einer kationogenen und/oder kationischen Gruppe pro Molekül,
d) 0,1 bis 30 Gew.-% wenigstens eines α,β-ethylenisch ungesättigten Monomers der allgemeinen Formel II 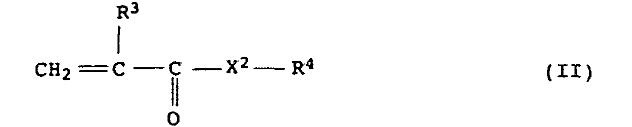 worin
R³ für Wasserstoff oder C₁- bis C₈-Alkyl steht,
X² für 0 oder NR⁵ steht, wobei R⁵ für wasserstoff, C₁- bis C₈-Alkyl oder C₅- bis C₈-Cycloalkyl steht, und
R⁴ für Wasserstoff oder einen linearen C₁- bis C₂₂-Alkylrest steht,
einpolymerisiert enthält.

11. Polymer nach Anspruch 10, das wenigstens eine weitere Komponente, die ausgewählt ist unter
e) von d) verschiedenen Verbindungen mit einer radikalisch polymerisierbaren α,β-ethylenisch ungesättigten Doppelbindung und mindestens einer anionogenen und/oder anionischen Gruppe pro Molekül,
f) vernetzend wirkenden Monomeren mit mindestens zwei ethylenisch ungesättigten, nicht konjugierten Doppelbindungen,
g) von a) bis f) verschiedenen radikalisch polymerisierbaren Monomeren,
und Mischungen davon, einpolymerisiert enthält.

12. Polymer nach einem der Ansprüche 10 oder 11, das 0,1 bis 20 Gew.-%, bezogen auf die Gesamtmenge der Monomere a), b), c) und d), wenigstens eines Monomers der allgemeinen Formel II mit X² = O und R⁴ = H einpolymerisiert enthält.

13. Polymer nach einem der Ansprüche 10 bis 12, das
10 bis weniger als 50 Gew.-% wenigstens eines Monomers der Formel I,
25 bis 70 Gew.-% wenigstens eines N-Vinylamids und/oder N-Vinyllactams,
3 bis 20 Gew.-% wenigstens einer Verbindung mit einer radikalisch polymerisierbaren, α,β-ethylenisch ungesättigten Doppelbindung und mindestens einer kationischen und/oder kationogenen Gruppe pro Molekül,
1 bis 10 Gew.-% wenigstens einer Verbindung der Formel II mit X² = O und R⁴ = H,
1 bis 20 Gew.-% wenigstens eines Polyalkylenoxidgruppen-haltigen Polysiloxans und gegebenenfalls
0 bis 25 Gew.-% einer oder mehrerer Verbindungen der Formel II mit R⁴ ≠ H, wenn X² = O,
einpolymerisiert enthält, wobei die Gewichtsanteile sich zu 100 Gew.-% addieren.

14. Verwendung der Polymere, wie in einem der Ansprüche 1 bis 7 definiert, als Hilfsmittel in der Kosmetik, bevorzugt als Beschichtungsmittel für Haar, Haut und Nägel, insbesondere in der Haarkosmetik, vorzugsweise als Festigerpolymer in Haarsprays, Schaumfestigern, Haarmousse, Haargel und Shampoos.

15. Verwendung der Polymere, wie in einem der Ansprüche 1 bis 7 definiert, in der dekorativen Kosmetik, bevorzugt in Mascara, Make-up und Lidschatten, als polymerer Emulgator oder Coemulgator zur Formulierung von kosmetischen oder pharmazeutischen Präparaten sowie als Hilfsmittel in der Pharmazie, bevorzugt als oder in Beschichtungsmittel(n) oder Bindemittel(n) für feste Arzneiformen.

16. Verwendung der Polymere, wie in einem der Ansprüche 1 bis 7 definiert, als oder in Beschichtungsmittel(n) für die Textil-, Papier-, Druck-, Leder- und Klebstoffindustrie sowie als Polymeremulgator für nicht-kosmetische Zubereitungen.

## Claims

1. A cosmetic composition comprising at least one water-soluble or water-dispersible polymer which comprises, in incorporated form,
a) 5 to less than 50% by weight of at least one α,β-ethylenically unsaturated monomer of the formula I 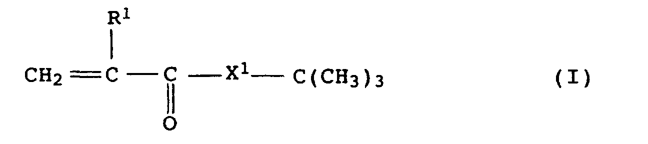 in which
R¹ is hydrogen or C₁- to C₈-alkyl, and
X¹ is O or NR², where R² is hydrogen, C₁- to C₈-alkyl or C₅- to C₈-cycloalkyl,
b) 25 to 90% by weight of at least one N-vinylamide and/or N-vinyllactam,
c) 0.5 to 30% by weight of at least one compound having a free-radically polymerizable, α,β-ethylenically unsaturated double bond and at least one cationogenic and/or cationic group per molecule,
d) 0 to 30% by weight of at least one α,β-ethylenically unsaturated monomer of the formula II 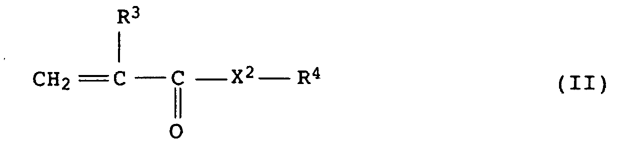 in which
R³ is hydrogen or C₁- to C₈-alkyl,
X² is O or NR⁵, where R⁵ is hydrogen C₁- to C₈-alkyl or C₅- to C₈-cycloalkyl, and
R⁴ is hydrogen or a linear C₁- to C₂₂-alkyl radical,
or the salts thereof.

2. A composition as claimed in claim 1, where the water-soluble or water-dispersible polymer comprises, in copolymerized form, at least one monomer d) of the formula II where X² = O and R⁴ = H in an amount of from 0.1 to 20% by weight, based on the total amount of the monomers a), b), c) and d).

3. A composition as claimed in either of the preceding claims, where the polymer comprises, in copolymerized form, at least one further component which is chosen from
e) compounds different from d) having a free-radically polymerizable α,β-ethylenically unsaturated double bond and at least one anionogenic and/or anionic group per molecule,
f) crosslinking monomers with at least two ethylenically unsaturated, nonconjugated double bonds,
g) free-radically polymerizable monomers different from a) to f),
and mixtures thereof.

4. A composition as claimed in any of the preceding claims, which additionally comprises at least one polysiloxane which contains polyalkylene oxide groups.

5. A composition as claimed in claim 4, where the polysiloxane is chosen from compounds of the formula III 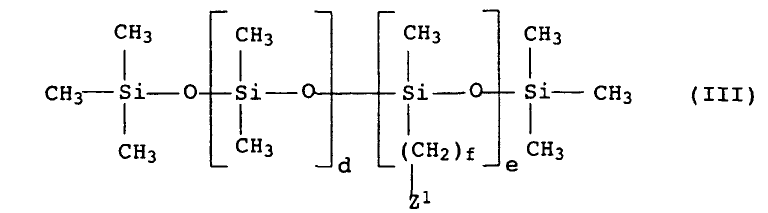 in which
the order of the siloxane units is arbitrary,
d and e, independently of one another, are 0 to 100, where the sum d + e is at least 2,
f is an integer from 2 to 8,
Z¹ is a radical of the formula IV
-(CH₂)ᵤ-O-(CH₂CH₂O)ᵥ(CH₂CH(CH₃)O)_{w}-R^{A} (IV)
where
in the formula IV the order of the alkylene oxide units is arbitrary,
u is an integer from 1 to 8,
v and w, independently of one another, are an integer from 0 to 200, where the sum v + w is > 0, and
R^{A} is hydrogen or C₁-C₆-alkyl.

6. A composition as claimed in claim 4 or 5, where the quantitative ratio of water-soluble or water-dispersible polymer to polysiloxane is in a range from 70:30 to 99.9:0.1, preferably 85:15 to 99:1.

7. A composition as claimed in any of claims 4 to 6, where the water-soluble or water-dispersible polymer used is a polymer obtained by free-radical polymerization of the monomers forming the polymer in the presence of at least one polysiloxane which contains polyalkylene oxide groups.

8. A composition as claimed in any of the preceding claims in the form of a hair-treatment composition, in particular in the form of a hairspray.

9. A composition as claimed in claim 8, comprising
A) 0.2 to 20% by weight of at least one water-soluble or water-dispersible polymer, as defined in any of claims 1 to 6,
B) 30 to 99.5% by weight, preferably 40 to 99% by weight, of at least one solvent chosen from water, water-miscible solvents and mixtures thereof,
C) 0 to 70% by weight of propellant,
D) 0 to 10% by weight of at least one water-soluble or water-dispersible hair polymer which is different from A),
E) 0 to 0.3% by weight of at least one water-insoluble silicone,
F) 0 to 0.5% by weight of at least one wax, preferably at least one fatty acid amide.

10. A water-soluble or water-dispersible polymer which comprises, in copolymerized form,
a) 5 to less than 50% by weight of at least one α,β-ethylenically unsaturated monomer of the formula I 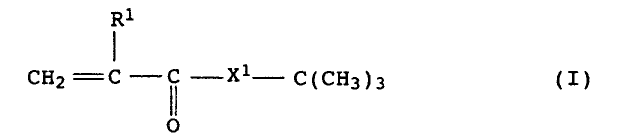 in which
R¹ is hydrogen or C₁- to C₈-alkyl, and
X¹ is O or NR², where R² is hydrogen, C₁- to C₈-alkyl or C₅- to C₈-cycloalkyl,
b) 25 to 90% by weight of at least one N-vinylamide and/or N-vinyllactam,
c) 0.5 to 30% by weight of at least one compound having a free-radically polymerizable, α,β-ethylenically unsaturated double bond and at least one cationogenic and/or cationic group per molecule,
d) 0.1 to 30% by weight of at least one α,β-ethylenically unsaturated monomer of the formula II 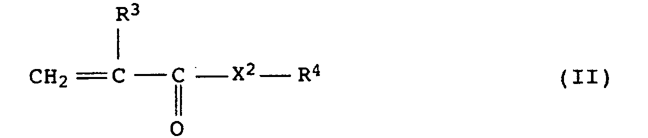 in which
R³ is hydrogen or C₁- to C₈-alkyl,
X² is O or NR⁵, in which R⁵ is hydrogen C₁- to C₈-alkyl or C₅- to C₈-cycloalkyl, and
R⁴ is hydrogen or a linear C₁- to C₂₂-alkyl radical.

11. A polymer as claimed in claim 10, which comprises, in copolymerized form, at least one further component which is chosen from
e) compounds different from d) having a free-radically polymerizable α,β-ethylenically unsaturated double bond and at least one anionogenic and/or anionic group per molecule,
f) crosslinking monomers with at least two ethylenically unsaturated, nonconjugated double bonds,
g) free-radically polymerizable monomers different from a) to f),
and mixtures thereof.

12. A polymer as claimed in either of claims 10 and 11 which comprises, in copolymerized form, 0.1 to 20% by weight, based on the total amount of monomers a), b), c) and d), of at least one monomer of the formula II where X² = O and R⁴ = H.

13. A polymer as claimed in one of claims 10 to 12, which comprises, in copolymerized form
10 to less than 50% by weight of at least one monomer of the formula I,
25 to 70% by weight of at least one N-vinylamide and/or N-vinyllactam,
3 to 20% by weight of at least one compound having a free-radically polymerizable, α,β-ethylenically unsaturated double bond and at least one cationic and/or cationogenic group per molecule,
1 to 10% by weight of at least one compound of the formula II where X² = O and R⁴ = H,
1 to 20% by weight of at least one polysiloxane which contains polyalkylene oxide groups, and optionally
0 to 25% by weight of one or more compounds of the formula II where R⁴ ≠ H, if X² = O,
where the weight fractions add up to 100% by weight.

14. The use of the polymers as defined in any of claims 1 to 7, as auxiliaries in cosmetics, preferably as coating compositions for hair, skin and nails, in particular in hair cosmetics, preferably as setting polymers in hair-sprays, setting foams, hair mousse, hair gel and shampoos.

15. The use of a polymer as defined in any of claims 1 to 7 in decorative cosmetics, preferably in mascara, foundation and eye shadows, as polymer emulsifier or coemulsifier for the formulation of cosmetic or pharmaceutical preparations, and as auxiliaries in pharmacy, preferably as or in coating composition(s) or binder(s) for solid medicament forms.

16. The use of the polymers as defined in any of claims 1 to 7, as or in coating composition(s) for the textile, paper, printing, leather and adhesive industries, and as polymer emulsifier for noncosmetic preparations.

## Revendications

1. Produit cosmétique contenant au moins un polymère soluble ou dispersable à l'eau, qui contient à l'état chimiquement combiné
a) de 5 à moins de 50% en poids d'au moins un monomère à insaturation α,β-éthylénique de formule générale I : 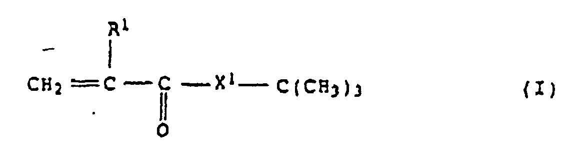 dans laquelle
R¹ représente l'hydrogène ou un groupe alkyle en C1-C8 et
X¹ représente O ou NR², R² représentant l'hydrogène, un groupe alkyle en C1-C8 ou cycloalkyle en C5-C8,
b) 25 à 90% en poids d'au moins un N-vinylamide et/ou N-vinyllactame,
c) 0,5 à 30% en poids d'au moins un composé ayant au moins une double liaison insaturée α,β-éthylénique apte à la polymérisation radicalaire et au moins un groupe cationogène et/ou cationique par molécule,
d) 0 à 30% en poids d'au moins un monomère à insaturation α,β-éthylénique de formule générale II : 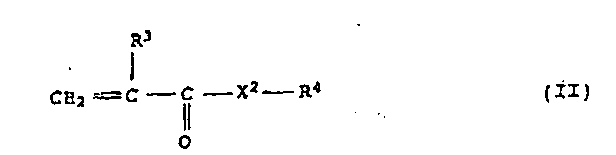 dans laquelle
R³ représente l'hydrogène ou un groupe alkyle en C1-C8,
X² représente O ou NR⁵, R⁵ représentant l'hydrogène, un groupe alkyle en C1-C8 ou cycloalkyle en C5-C8, et
R⁴ représente l'hydrogène ou un groupe alkyle à chaîne droite en C1-C22,
et ses sels.

2. Produit selon la revendication 1, dans lequel le polymère soluble ou dispersable à l'eau contient au moins un monomère d) de formule II dans laquelle X² = 0 et R⁴ = H en proportions de 0,1 à 20% du poids total des monomères a), b), c) et d), à l'état polymérisé.

3. Produit selon l'une des revendications qui précèdent dans lequel le polymère contient au moins un autre composant choisi parmi :
e) des composés, autres que ceux mentionnés en d), contenant une double liaison insaturée α,β-éthylénique apte à la polymérisation radicalaire et au moins un groupe anionogène et/ou anionique par molécule,
f) des monomères réticulants à au moins deux doubles liaisons insaturées éthyléniques non conjuguées,
g) des monomères aptes à la polymérisation radicalaire, autres que ceux mentionnés en a) à f),
et leurs mélanges, à l'état polymérisé.

4. Produit selon l'une des revendications qui précèdent, contenant en outre au moins un polysiloxane qui contient des groupes oxyde de polyalkylène.

5. Produit selon la revendication 4, pour lequel le polysiloxane est choisi parmi les composés qui répondent à la formule générale III : 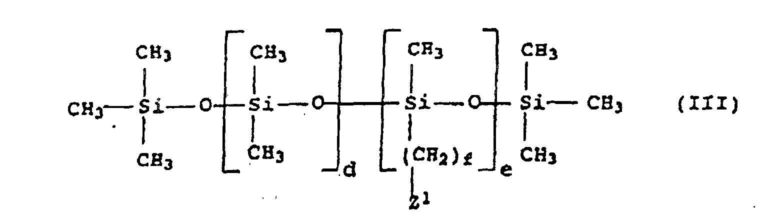 dans laquelle les motifs de siloxane sont dans un ordre quelconque,
d et e sont chacun, indépendamment l'un de l'autre, un nombre de 0 à 100,
la somme de d et e étant égale au moins à 2,
f est un nombre entier allant de 2 à 8,
Z¹ représente un groupe de formule IV
-(CH₂)ᵤ-O-(CH₂CH₂O)ᵥ(CH₂CH(CH₃)O)_{w}-R^{A} (IV)
dans laquelle
l'ordre des motifs d'oxyde d'alkylène est quelconque,
u est un nombre entier allant de 1 à 8,
v et w sont chacun, indépendamment l'un de l'autre, un nombre entier allant de 0 à 200, la somme de v et w étant supérieure à 0, et
R^{A} représente l'hydrogène ou un groupe alkyle en C1-C6.

6. Produit selon l'une des revendications 4 ou 5, dans lequel les proportions relatives entre le polymère soluble ou dispersable à l'eau et le polysiloxane se situent dans l'intervalle de 70: 30 à 99,9: 0,1, de préférence de 85 : 15 à 99 : 1.

7. Produit selon l'une des revendications 4 à 6, pour lequel on utilise en tant que polymère soluble ou dispersable à l'eau un polymère obtenu par polymérisation radicalaire des monomères constituants en présence d'au moins un polysiloxane à groupes oxyde de polyalkylène.

8. Produit selon l'une des revendications qui précèdent, sous forme d'un produit pour le traitement de la chevelure, en particulier sous forme d'un aérosol capillaire.

9. Produit selon la revendication 8, contenant
A) 0,2 à 20% en poids d'au moins un polymère soluble ou dispersable à l'eau tel que défini dans l'une des revendications 1 à 6,
B) 30 à 99,5% en poids, de préférence 40 à 99% en poids d'au moins un solvant choisi parmi l'eau, les solvants miscibles à l'eau et leurs mélanges,
C) 0 à 70% en poids d'un propulseur,
D) 0 à 10% en poids d'au moins un polymère capillaire, soluble ou dispersable à l'eau, autre que A),
E) 0 à 0,3% en poids d'au moins un silicone insoluble dans l'eau,
F) 0 à 0,5% en poids d'au moins une cire, de préférence au moins un amide d'acide gras.

10. Polymère soluble ou dispersable à l'eau contenant, à l'état polymérisé,
a) de 5 à moins de 50% en poids d'au moins un monomère à insaturation α,β-éthylénique de formule générale I : 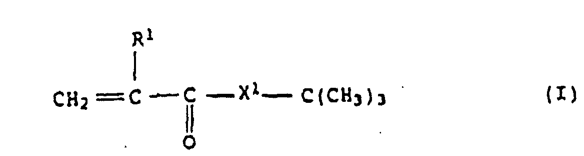 dans laquelle
R¹ représente l'hydrogène ou un groupe alkyle en C1-C8, et
X¹ représente O ou NR2, R2 représentant l'hydrogène, un groupe alkyle en C1-C8 ou cycloalkyle en C5-C8,
b) 25 à 90% en poids d'au moins un N-vinylamide et/ou N-vinyllactame,
c) 0,5 à 30% en poids d'au moins un composé contenant une double liaison insaturée α,β-éthylénique apte à la polymérisation radicalaire et au moins un groupe cationogène et/ou cationique par molécule,
d) 0,1 à 30% en poids d'au moins un monomère à insaturation α,β-éthylénique de formule générale II : 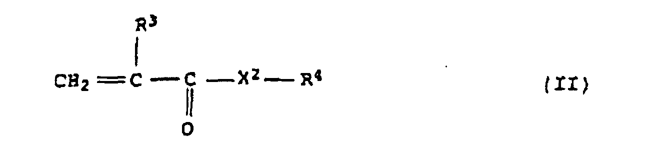 dans laquelle
R³ représente l'hydrogène ou un groupe alkyle en C1-C8,
X² représente O ou NR⁵, R⁵ représentant l'hydrogène, un groupe alkyle en C1-C8 ou cycloalkyle en C5-C8 et
R⁴ représente l'hydrogène ou un groupe alkyle à chaîne droite en C1-C22.

11. Polymère selon la revendication 10, contenant, à l'état polymérisé, au moins un autre composant choisi parmi :
e) des composés, autre que ceux mentionnés en d), contenant une double liaison insaturée α,β-éthylénique apte à la polymérisation radicalaire et au moins un groupe anionogène et/ou anionique par molécule,
f) des monomères réticulants à au moins deux doubles liaisons à insaturation éthylénique non conjuguées,
g) des monomères aptes à la polymérisation radicalaire, autres que ceux mentionnés en a) à f),
et leurs mélanges.

12. Polymère selon l'une des revendications 10 ou 11, contenant à l'état polymérisé, 0,1 à 20% en poids, par rapport au poids total des monomères a), b), c) et d), d'au moins un monomère de formule générale II dans laquelle X² = O et R⁴ = H.

13. Polymère selon l'une des revendications 10 à 12, contenant, à l'état polymérisé,
- de 10 à moins de 50% en poids d'au moins un monomère de formule I,
- de 25 à 70% en poids d'au moins un N-vinylamide et/ou N-vinyllactame,
- de 3 à 20% en poids d'au moins un composé contenant une double liaison insaturée α,β-éthylénique apte à la polymérisation radicalaire et au moins un groupe cationique et/ou cationogène par molécule,
- de 1 à 10% en poids d'au moins un composé de formule II dans laquelle X² = O et R⁴ = H,
- de 1 à 20% en poids d'au moins un polysiloxane contenant des groupes oxyde de polyalkylène et le cas échéant
- de 0 à 25% en poids d'un ou plusieurs composés de formule II dans laquelle R⁴ a une signification autre que H lorsque X² = O,
ces pourcentages en poids formant un total de 100% en poids.

14. Utilisation des polymères tels que définis dans l'une des revendications 1 à 7 en tant que produits auxiliaires en cosmétique, de préférence en tant que produits de revêtement pour la chevelure, la peau et les ongles, et en particulier en cosmétique capillaire, de préférence en tant que polymères fixateurs dans des aérosols capillaires, des fixateurs en mousse, des mousses capillaires, des gels capillaires et des shampooings.

15. Utilisation des polymères tels que définis dans l'une des revendications 1 à 7 en cosmétique décorative, de préférence dans des mascaras, des maquillages et des fards à paupières, en tant qu'agents émulsionnants polymères ou agents émulsionnants auxiliaires pour la formulation de compositions cosmétiques ou pharmaceutiques et en tant que produits auxiliaires en pharmacie, de préférence en tant que produits de revêtements ou dans des produits de revêtement ou liants pour formes pharmaceutiques solides.

16. Utilisation des polymères définis dans l'une des revendications 1 à 7 en tant que produits de revêtement ou dans des produits de revêtement pour l'industrie textile, l'industrie du papier, l'impression, l'industrie du cuir et l'industrie des adhésifs ainsi qu'en tant qu'agents émulsionnants polymères pour des compositions non cosmétiques.
